(19) Europäisches Patentamt
European Patent Office
Office européen des brevets

(11) EP 1 270 738 A1

(12) # EUROPÄISCHE PATENTANMELDUNG

(43) Veröffentlichungstag:
**02.01.2003 Patentblatt 2003/01**

(51) Int Cl.⁷: **C12Q 1/68**, G01N 33/53

(21) Anmeldenummer: **01114562.0**

(22) Anmeldetag: **18.06.2001**

(84) Benannte Vertragsstaaten:
**AT BE CH CY DE DK ES FI FR GB GR IE IT LI LU MC NL PT SE TR**
Benannte Erstreckungsstaaten:
**AL LT LV MK RO SI**

(71) Anmelder: **Chimera Biotec GmbH**
**28209 Bremen (DE)**

(72) Erfinder:
- **Niemeyer, Christof M., Dr.**
  **28213 Bremen (DE)**
- **Wacker, Ron**
  **28215 Bremen (DE)**
- **Adler, Michael**
  **27607 Langen-Debstedt (DE)**

(74) Vertreter: **Eisenführ, Speiser & Partner**
**Martinistrasse 24**
**28195 Bremen (DE)**

(54) **Verfahren zum Nachweis von Substanzen in Flüssigkeiten**

(57) Die Erfindung betrifft ein Verfahren zum Nachweisen einer Substanz in wässriger Lösung. Dabei wird die nachzuweisende Substanz mit einer Nachweis-Nucleinsäure gekuppelt, die in einem Folgeschritt in Gegenwart einer Kompetitor-Nucleinsäure amplifiziert wird. Durch die kompetetive co-Amplifikation von Marker und Kompetitor-Nucleinsäure und der nachfolgenden Berechnung des Quotienten der für die jeweiligen Amplifikationsprodukte erhalten Signale eines Nucleinsäure-Nachweises wird eine hochsenstive Detektion der nachzuweisenden Substanz, insbesondere aus biologischer Matrix, ermöglicht.

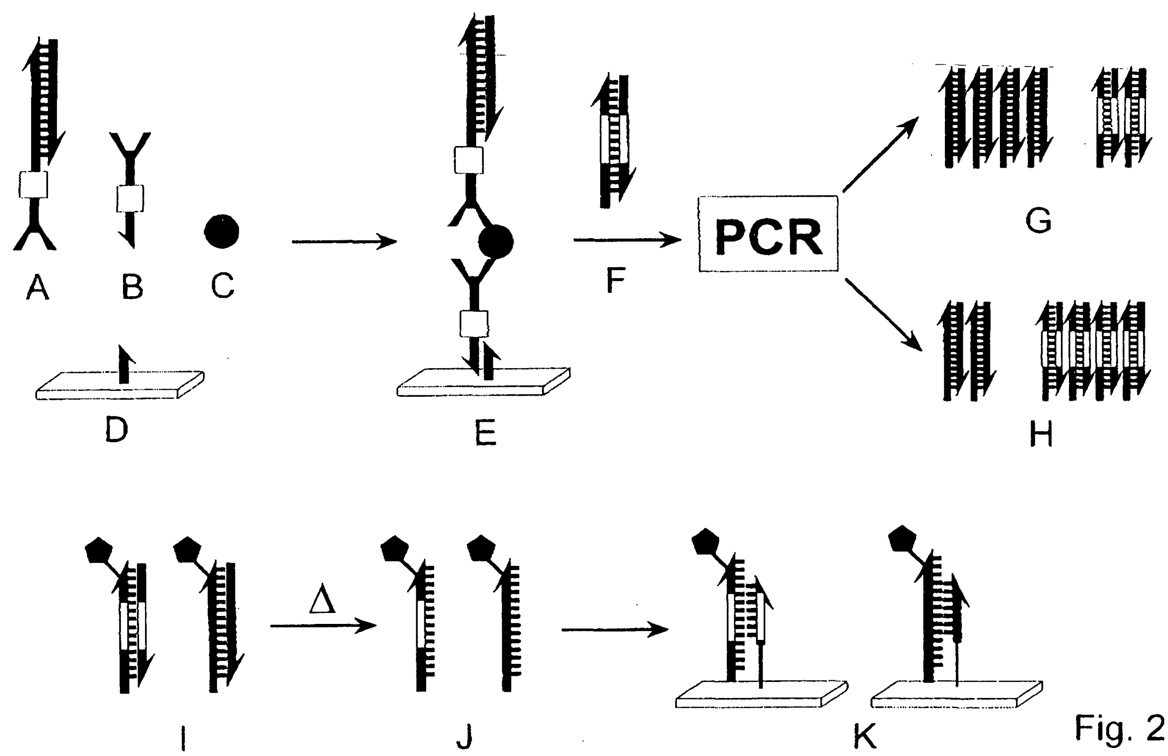


Fig. 2

EP 1 270 738 A1

SEQUENZPROTOKOLL

<110> chimera biotec GmbH

<120> Verfahren zum Nachweis von Substanzen in Flüssigkeiten

<130> C 821

<140>
<141>

<160> 15

<170> PatentIn Ver. 2.1

<210> 1
<211> 24
<212> DNA
<213> Künstliche Sequenz

<220>
<223> Beschreibung der künstlichen Sequenz:Primer N-1

<400> 1
agcggataac aatttcacac agga 24

<210> 2
<211> 21
<212> DNA
<213> Künstliche Sequenz

<220>
<223> Beschreibung der künstlichen Sequenz:Primer N-2

<400> 2
aaggcgatta agttggaaaa c 21

<210> 3
<211> 45
<212> DNA
<213> Künstliche Sequenz

<220>
<223> Beschreibung der künstlichen Sequenz:Primer K-1

<400> 3

1

agcggataac aatttcacac aggacgctca tgagacataa ccctg 45

<210> 4
<211> 64
<212> DNA
<213> Künstliche Sequenz

<220>
<223> Beschreibung der künstlichen Sequenz:Primer K-2

<400> 4
aaggcgatta agttgggtaa cgccatgagc gttcgtggga tagtcaaaat gccgcaaaaa 60
aggg 64

<210> 5
<211> 16
<212> DNA
<213> Künstliche Sequenz

<220>
<223> Beschreibung der künstlichen Sequenz:Primer H-1

<400> 5
tgcagcctcc aggacc 16

<210> 6
<211> 18
<212> DNA
<213> Künstliche Sequenz

<220>
<223> Beschreibung der künstlichen Sequenz:Primer H-2

<400> 6
gcactcgcaa gcacccta 18

<210> 7
<211> 21
<212> DNA
<213> Künstliche Sequenz

<220>
<223> Beschreibung der künstlichen Sequenz:Fänger-Oligonucleotid cN-Es

<220>
<221> modified_base
<222> (1)
<223> biotinyliert

<400> 7
gtaatcatgg tcatagctgt t 21

<210> 8
<211> 19
<212> DNA
<213> Künstliche Sequenz

<220>
<223> Beschreibung der künstlichen
Sequenz:Fänger-Oligonucleotid cK-Es

<220>
<221> modified_base
<222> (1)
<223> biotinyliert

<400> 8
actatcccac gaacgctca 19

<210> 9
<211> 24
<212> DNA
<213> Künstliche Sequenz

<220>
<223> Beschreibung der künstlichen
Sequenz:Kupplungs-Sequenz bcA

<220>
<221> modified_base
<222> (1)
<223> biotinyliert

<400> 9
agcggataac aatttcacac agga 24

<210> 10
<211> 24

<212> DNA
<213> Künstliche Sequenz

<220>
<223> Beschreibung der künstlichen Sequenz:Kupplungs-Sequenz A

<400> 10
tcctgtgtga aattgttatc cgct 24

<210> 11
<211> 21
<212> DNA
<213> Künstliche Sequenz

<220>
<223> Beschreibung der künstlichen Sequenz:Erkennungssequenz N-Es

<400> 11
aacagctatg accatgatta c 21

<210> 12
<211> 19
<212> DNA
<213> Künstliche Sequenz

<220>
<223> Beschreibung der künstlichen Sequenz:Erkennungssequenz K-Es

<400> 12
tgagcgttcg tgggatagt 19

<210> 13
<211> 169
<212> DNA
<213> Künstliche Sequenz

<220>
<223> Beschreibung der künstlichen Sequenz:Nachweis-Nucleinsäure "DNA-169" - Position 6252-6354 auf M13mp18

<220>

<221> modified_base
<222> (1)
<223> biotinyliert

<220>
<221> modified_base
<222> (169)
<223> biotinyliert

<220>
<221> primer_bind
<222> (1)..(17)
<223> Bindungsstelle für Primer "N-2"

<220>
<221> primer_bind
<222> (146)..(169)
<223> Bindungsstelle für Primer "N-1"

<220>
<221> misc_binding
<222> (125)..(145)
<223> Erkennungssequenz "N-Es"

<400> 13
aaggcgatta agttgggtaa cgccagggtt ttcccagtca cgacgttgta aaacgacggc 60
cagtgccaag cttgcatgcc tgcaggtcga ctctagagga tccccgggta ccgagctcga 120
attcgtaatc atggtcatag ctgtttcctg tgtgaaattg ttatccgct 169

<210> 14
<211> 177
<212> DNA
<213> Künstliche Sequenz

<220>
<223> Beschreibung der künstlichen
Sequenz:Kompetitor-Nucleinsäure ("KF1" -
artifiziell) mit den Primerbindungsstellen "N-2"
und "N-1" der Nachweis-Nucleinsäure

<220>
<221> primer_bind
<222> (1)..(17)
<223> BindungsstellePrimer "N-2"

<220>
<221> primer_bind

<222> (154)..(177)
<223> Bindungsstelle Primer "N-1"

<220>
<221> misc_binding
<222> (26)..(44)
<223> Erkennungssequenz "K-Es"

<400> 14
aaggcgatta agttgggtaa cgccatgagc gttcgtggga tagtcaaaat gccgcaaaaa 60
agggaataag ggcgacacgg aaatgttgaa tactcatact cttccttttt caatattatt 120
gaagcattta tcagggttat tgtctcatga gcgtcctgtg tgaaattgtt atccgct 177

<210> 15
<211> 209
<212> DNA
<213> Künstliche Sequenz

<220>
<223> Beschreibung der künstlichen
Sequenz:Kompetitor-Nucleinsäure "KF-2"

<220>
<221> primer_bind
<222> (1)..(16)
<223> Bindungsstelle Primer "H-1"

<220>
<221> primer_bind
<222> (192)..(209)
<223> Bindungsstelle Primer "H-2"

<220>
<221> misc_binding
<222> (1)..(16)
<223> Erkennungssequenz, identisch mit Bindungssequenz
für Primer "H-1"

<400> 15
tgcagcctcc aggacccccc ctcccgggag agccatagtg gtctgcggaa ccggtgagta 60
caccggaatt gccaggacga ccgggtcctt tcttggatta acccgctcaa tgcctggaga 120
tttgggcgtg cccccgcgag actgctagcc gagtagtgtt gggtcgcgaa aggccttgtg 180
gtactgcctg atagggtgct tgcgagtgc 209

**Beschreibung**

Die Erfindung betrifft ein Verfahren zum Nachweis einer Substanz (Analyt), vorzugsweise in wässriger Lösung.

Die Einführung von Antikörper- oder Rezeptor-basierten Immunoassays in den 60er und 70er Jahren führte zur Entwicklung einer Vielzahl hochspezifischer Verfahren, um niedrigkonzentrierte Analytsubstanzen empfindlich und präzise nachzuweisen. So beschreibt J.R. Crowther in ELISA: Theory and Practice, Humana Press Inc., 1995, dass mit Enzym-verstärkten Assays oder Radioimmuno-Assays Substanzen mit Konzentrationen bis in den Attomol-Bereich (1 amol=1 $\times$ $10^{-18}$ mol) nachgewiesen werden können. Die Entwicklung hochwirksamer Wirkstoffe und Behandlungsmethoden der modernen Medizin, aber auch das Auftreten neuer Krankheiten wie die über das Prionprotein $PrP^{Sc}$ nachweisbaren Encephalopathien (beispielsweise die bovine spongiforme Encephalopathie, Scrapie, Creutzfeld-Jacob-Krankheit, Gerstmann-Straussler-Scheinker-Syndrom) erfordern jedoch die Entwicklung von Nachweisverfahren, um niedrigstkonzentrierte Substanzen, möglichst im Subattomol- oder Zeptomolbereich (1 zmol=1 $\times$ $10^{-21}$ mol), nachzuweisen.

Ferner besteht ein generelles, bislang ungelöstes Problem darin, niedrig- und niedrigstkonzentrierte Analytsubstanzen aus komplexen Medien wie Blutplasma und - serum mit hoher Empfindlichkeit und Sicherheit nachzuweisen.

C.M. Niemeyer et al., *Nucleic Acids Res.* 1999, 4553 bis 4561, beschreiben ein Nachweisverfahren, bei dem die nachzuweisende (Nicht-Nucleinsäure-) Substanz mit einem zuvor gebildeten supramolekularen Nachweis-Reagens markiert wird, wobei das Nachweis-Reagens ein Komplex aus durch Streptavidin oligomerisierten biotinylierten DNA-Markerfragmenten und biotinylierten Antikörpern gegen die nachzuweisende Substanz ist. Die im Nachweis-Reagens mit der nachzuweisenden Substanz verknüpften DNA-Markerfragmente wurden durch PCR (polymerase chain reaction) nachgewiesen. Verfahren dieser Art können als "Immuno-PCR" bezeichnet werden. Im Vergleich zu einem herkömmlichen ELISA konnte die Nachweisgrenze um etwa den Faktor 1000 gesenkt werden. Das Verfahren ist jedoch noch immer nur schwer handhabbar und nicht empfindlich genug. Auch die darin beschriebenen Waschschritte sind wegen der damit verbundenen Einbußen in der Nachweisempfindlichkeit nachteilig. Ebenfalls ist nachteilig, dass es im PCR-Nachweis zu zufälligen, kaum vorhersagbaren oder steuerbaren Schwankungen in der Amplifikationseffizienz und dadurch auch in der Endkonzentration der amplifizierten DNA-Markerfragmente kommt. Diese zufälligen Schwankungen in der Endkonzentration der DNA-Markerfragmente verhindern eine zuverlässige Quantifizierung der nachzuweisenden Substanz. Dies ist insbesondere der Fall bei niedrigkonzentrierten nachzuweisenden Substanzen (deren Konzentration bspw. nur etwa $10^{-21}$ mol oder weniger beträgt). Besonders nachteilig wirkt sich dies beim Nachweis von Substanzen in komplexen biologischen Proben (biologische Matrizes) wie Blut, Blutserum und dessen Bestandteile, Körperflüssigkeiten wie Speichel, Urin, Sperma, Rückenmarksflüssigkeit oder Aufschlüsse aus biologischem Material wie Zell- oder Gewebeaufschlüsse aus.

Es war eine Aufgabe der Erfindung, ausgehend von dem vorgenannten Stand der Technik ein weiteres Verfahren zum Nachweisen niedrigstkonzentrierter Substanzen anzugeben. Mit dem Nachweisverfahren sollte vorzugsweise nicht nur das Vorliegen und/oder die Abwesenheit einer Substanz nachgewiesen werden können (qualitativer Nachweis), sondern auch die Menge und/oder Konzentration der nachzuweisenden Substanz bestimmbar sein (quantitativer Nachweis).

Es war eine weitere Aufgabe, ein Nachweisverfahren anzugeben, das mit möglichst wenigen Waschschritten durchgeführt werden kann.

Eine weitere Aufgabe war es, ein Nachweisverfahren anzugeben, das zum Nachweis möglichst vieler unterschiedlicher nachzuweisender Substanzen, beispielsweise Proteine, Peptide, Zucker, Lipide und dergleichen, einsetzbar ist.

Die genannten Aufgaben werden gelöst durch ein Verfahren zum Nachweisen einer Substanz in einem Testansatz, umfassend die Schritte:

a) Bilden eines Nachweiskomplexes im Testansatz, wobei im Nachweiskomplex

- die nachzuweisende Substanz an ein Nachweis-Bindungsreagens gebunden ist und
- das Nachweis-Bindungsreagens mit einer Nachweis-Nucleinsäure, die mit einem Replikations-Reagens replizierbar (amplifizierbar) ist, zu einem Nachweiskonjugat verbunden ist,

b) Zugeben einer von der Nachweis-Nucleinsäure unterscheidbaren Kompetitor-Nucleinsäure zum Testansatz, wobei die Kompetitor-Nucleinsäure ebenfalls durch das Replikations-Reagens replizierbar ist und mit der Nachweis-Nucleinsäure um mindestens einen Bestandteil des Replikations-Reagens konkurriert,

c) Kontaktieren der Nachweis- und Kompetitor-Nucleinsäuren mit dem Replikations-Reagens zum Replizieren der Nachweis- und Kompetitor-Nucleinsäuren,

d) Nachweisen der replizierten Nachweis-Nucleinsäure und der replizierten Kompetitor-Nucleinsäure.

Aus G. Gilliland, Analysis of cytokine mRNA and DNA: Detection and quantitation by competitive polymerase chain reaction, *Proc. Natl. Acad. Sci.*, 1990: 2725-2729 ist ein Verfahren zur Quantifizierung niedrig konzentrierter DNA bekannt. Die zu quantifizierende DNA und eine Kompetitor-DNA werden gemeinsam repliziert (amplifiziert). Dazu umfasst das Replikations-Reagens Oligonucleinsäuren, mit denen sowohl die Nachweis-Nucleinsäure als auch die Kompetitor-Nucleinsäure replizierbar sind. Aus dem Verhältnis der gebildeten PCR-Produkte (replizierte zu quantifizierende DNA und replizierte Kompetitor-DNA) kann die Ausgangskonzentration der zu quantifizierenden DNA berechnet werden.

Dieses bekannte Verfahren ist auf die Quantifizierung von Nucleinsäuren beschränkt und lässt sich nicht auf die Quantifizierung von Nicht-Nudeinsäuren übertragen. Hinzu kommt, dass PCR-Verfahren sehr empfindlich auf Verunreinigungen wie Proteine, Peptide und andere nachzuweisende oder zu quantifizierende Substanzen reagieren (siehe dazu auch C.R. Newton, A. Graham, "PCR" (Labor im Focus), Spektrum Akademischer Verlag, 2. Auflage 1994). Deshalb war zu erwarten, dass sich das Verfahren nur eingeschränkt oder gar nicht zum Nachweis oder gar zur Quantifizierung von Nicht-Nucleinsäuren anwenden lässt. Hinzu kommt, dass es bei PCR-Verfahren notwendig ist, das Gefäß, in dem die PCR-Reaktion durchgeführt werden soll, möglichst frei von solchen Nucleinsäuren zu halten, die nicht repliziert werden sollen. Dies ist jedoch bei der Immuno-PCR nicht möglich: Entweder müssen zur Markierung der nachzuweisenden Substanz fertige Nachweiskonjugate (die Nachweis-Nucleinsäuren umfassen) zum Testansatz zugegeben werden, wobei nicht an die nachzuweisende Substanz gebundene Nachweiskonjugate nachträglich wieder entfernt werden müssen, oder die Nachweiskonjugate müssen erst hergestellt werden, wozu Nachweis-Nucleinsäuren zum Testansatz zugegeben werden müssen und entsprechend nicht in Nachweis-Komplexen vorliegende Nachweis-Nucleinsäuren nachträglich entfernt werden müssen. In beiden Fällen kann jedoch nicht davon ausgegangen werden, dass die zu entfernende Nachweis-Nucleinsäure tatsächlich vollständig entfernt wird. Dementsprechend war zu erwarten, dass eine Immuno-PCR zu hohen Hintergrundsignalen (in Form von replizierter Nachweis-Nucleinsäure ohne Vorliegen der nachzuweisenden Substanz) führt.

Überraschenderweise hat sich jedoch in eigenen Untersuchungen herausgestellt, dass sich mit dem erfindungsgemäßen Verfahren eine Vielzahl von Substanzen, insbesondere auch Nicht-Nucleinsäuren, nachweisen und quantifizieren lassen. Mit dem erfindungsgemäßen Verfahren konnten Substanzen in ähnlich niedrigen Konzentrationen oder sogar In geringeren Konzentrationen nachgewiesen und quantifiziert werden wie mit herkömmlichen Verfahren (beispielsweise ELISA).

Zu den Substanzen, die mit dem erfindungsgemäßen Verfahren nachgewiesen und/oder quantifiziert werden können, zählen insbesondere Proteine, Peptide, Glykoproteine, Kohlenhydrate, Lipide und andere Makromoleküle, aber auch niedermolekulare Verbindungen wie Pharmakophore, Drogen, Dopingmittel, Hormone oder andere Wirkstoffe oder Metabolite.

Im Sinne des erfindungsgemäßen Verfahrens werden solche Substanzen oder Substanzgemische als Bindungsreagenzien bezeichnet, die unter den gewählten Verfahrensbedingungen vorzugsweise selektiv an die nachzuweisen Substanz binden können. Bindungsreagenzien können insbesondere Rezeptoren wie Protein A und G und Enzyme sein, aber auch mono- und/oder polyclonale Immunglobuline, insbesondere solche vom Typ G, und deren Fragmente sein. Solche Fragmente sind insbesondere bekannt unter den Bezeichnungen Fab, $F(ab)_2$, dsFv-Fragmente, scFv-Fragmente und Single-Chain-Antikörper. Des weiteren können Bindungsreagenzien auch niedermolekulare Stoffe sein wie Peptide, Peptoide, Haptene und Nucleinsäure-Bindungsreagenzien wie Aptamere. Die Verwendung polyclonaler Immunglobuline als Bindungsreagenzien ist wegen ihrer im Vergleich zu monoclonalen Immunglobulinen häufig höheren Stabilität und häufig leichteren Verfügbarkeit bevorzugt. Wenn das Bindungsreagens ein Substanzgemisch mehrerer Substanz-Varianten wie beispielsweise ein polyclonales Immunglobulin ist, so umfasst das Nachweiskonjugat zumindest eine Variante aus der Gesamtzahl der das Bindungsreagens ausmachenden Substanz-Varianten (beispielsweise eine der Varianten eines polyclonalen Immunglobulins).

Nucleinsäuren (insbesondere Nachweis- und Kompetitor-Nucleinsäuren) im Sinne des Nachweisverfahrens sind Substanzen, die eine oder mehrere Nucleobasen (Adenin, Cytosin, Guanin, Thymin, Uracil) oder deren funktionale Analoga, wie zum Beispiel Hypoxanthin, umfassen. Die Nucleobasen sind dabei vorzugsweise mit einem Zucker, insbesondere mit einer Pentose, Pentopyranose oder Pentofuranose verbunden. Besonders bevorzugt sind dabei Ribose und Desoxyribose. Die Zucker wiederum sind vorzugsweise über Phosphodiester-Bindungen miteinander verknüpft. Der Begriff Nucleinsäure umfasst auch sogenannte Peptid-Nucleinsäuren, bei denen das Zuckerphosphat-Rückgrat durch Aminosäuren, die durch Peptidbindungen miteinander verbunden sind, ersetzt ist (PNAs, siehe dazu beispielsweise E. Uhlmann, *Biol. Chem.* 1998, 1045-1052), und Pyranosyl-Nucleinsäuren wie p-RNAs (sie dazu beispielsweise DE-A-19741716), sowie Alanylpeptidnucleinsäuren (Diederichsen, U, Paarungseigenschaften von Alanyl-Peptid-Nucleinsäuren mit alternierend konfigurierten Aminosäureeinheiten als Rückgrat. *Angew. Chem.* 1996, 458 bis 461) und Phosphothioat-Nucleinsäuren. Ein besonders vorteilhaftes Merkmal von Nucleinsäuren ist, dass diese chemisch, biochemisch oder biologisch vervielfältigt (repliziert, amplifiziert) werden können.

Das Nachweis-Bindungsreagens ist bzw. wird im erfindungsgemäßen Nachweisverfahren mit der Nachweis-Nucleinsäure zu einem Nachweiskonjugat verbunden. Bevorzugt ist es dabei, wenn sich die Verbindungen unter den gewählten Verfahrensbedingungen im wesentlichen nicht lösen, das heißt, dass während der Durchführung des Verfahrens weniger als 25% der Bindungen zerfallen.

Vorzugsweise erfolgt die Bindung über Biotin-Streptavidin-Bindungen (siehe dazu beispielsweise Niemeyer, C.M., Adler, M., Gao, F., Chi, L., *Bioconjugate Chemistry*, "Nanostructured DNA-Protein Networks Consisting of Covalent Oligonucleotide-Streptavidine Conjugates" Bioconjug Chem 12, 364-371, oder Diamandis, E. P. und T. K. Christopoulos (1991) "The biotin-(strept)avidin system: principles and applications in biotechnology." *Clin Chem* 37(5); 625-36). Besonders gute Ergebnisse lassen sich erzielen, wenn ein- oder mehrfach, vorzugsweise zwei- bis vierfach biotinylierte Nachweis-Bindungsreagenzien durch Streptavidin mit einoder mehrfach, vorzugsweise zweifach biotinylierten Nachweis-Nucleinsäuren verbunden werden. Gute Ergebnisse können ebenfalls erzielt werden, wenn die Nachweis-Nucleinsäure kovalent an Streptavidin gebunden ist und lediglich das Nachweis-Bindungsreagens biotinyliert ist. Vorzugsweise sollten freie Biotin-Bindungsstellen von Streptavidin durch Zugabe von Biotin abgesättigt werden, um störende Nebenreaktionen zu vermeiden.

Statt einer Biotin-Streptavidin-Bindung der am Nachweiskonjugat beteiligten Substanzen (Bindungsreagens, Nudeinsäuren) können diese auch auf andere Weise miteinander verbunden werden. Insbesondere können sie über kovalente Bindungen miteinander verbunden werden. Die Verwendung von Biotin-Streptavidin-Verbindungen ist jedoch bevorzugt, da Biotin und Streptavidin in hoher Reinheit zu geringem Preis und in großer Menge verfügbar sind, die Verknüpfung beider Substanzen mit einer Vielzahl von Stoffen, insbesondere von Nucleinsäuren und Immunglobulinen und Proteinen, leicht möglich ist und weil die Biotin-Streptavidin-Bindung unter üblichen Verfahrensbedingungen sehr stabil und leicht handhabbar ist.

Das Nachweiskonjugat kann bereits hergestellt werden, bevor die nachzuweisende Substanz an das Nachweis-Bindungsreagens bindet. Auf diese Weise können Konjugate aus Nachweis-Bindungsreagens und Nachweis-Nucleinsäuren bereits vor Durchführung eines Nachweisverfahrens vorgefertigt werden. Die Verbindung von Nachweis-Bindungsreagens und Nachweis-Nucleinsäure kann jedoch auch während oder nach der Bindung des Analyten (der nachzuweisenden Substanz) an das Nachweis-Bindungsreagens erfolgen.

Die Kompetitor-Nucleinsäure ist eine Nucleinsäure, die von der Nachweis-Nucleinsäure unterscheidbar ist, beispielsweise anhand ihrer Länge, ihrer Nucleobasen-Sequenz oder aufgrund von Markierungen wie beispielsweise Fluorophoren oder Radionucliden. Zur Unterscheidbarkeit von Nachweis- und Kompetitor-Nucleinsäure ist es jedoch ausreichend, wenn diese sich in einer Nucleobase unterscheiden. Vorzugsweise unterscheiden sich Nachweis- und Kompetitor-Nucleinsäure darin, dass in einer von beiden eine Schnittstelle für ein Restriktionsenzym vorliegt. Nach der Replikation können dann beide Nudeinsäuren anhand der Nukleinsäure-Fragmentgrößen, die sie bei einem Verdau durch dieses Restriktionsenzym hervorrufen, leicht unterschieden werden.

Besonders bevorzugt ist ein Verfahren, bei dem Nachweis- und Kompetitor-Nucleinsäuren unterschiedliche Erkennungssequenzen enthalten, anhand derer sie durch spezifische Nucleinsäure-Hybridisierung mit einem komplementären Fänger-Motiv erkannt und/oder voneinader getrennt werden können. Eine Erkennungssequenz ist dabei vorzugsweise ein Nucleinsäure-Motiv von 15-40 Basen Länge, deren komplementäre Sequenz diese mit einer hohen Hybridisierungseffizienz bindet, ohne dabei unspezifisch an weitere im Assay enthaltene Nucleinsäuren zu binden. Es ist ebenfalls bevorzugt, wenn die Hybridisierungseffizient der Erkennungssequenz der Nachweis- und Kompetitor-Nucleinsäure einander ähnlich sind, um störende Einflüsse unterschiedlicher Hybridisierungseffizienzen auf die Signalintensitäten beim Nachweis der Amplifikate von Nachweis-und Kompetitor-Nucleinsäure vorteilhaft gering zu halten.

Die Kompetitor-Nucleinsäure kann gemeinsam mit der Nachweis-Nucleinsäure durch das Replikations-Reagens (dazu siehe unten) repliziert werden. Dabei konkurrieren Nachweis- und Kompetitor-Nucleinsäure um zumindest einen Bestandteil des Replikations-Reagens; dieser Bestandteil limitiert die Replikations-Effizienzen beider Nucleinsäuren. Während bei üblichen Simultanreaktionen, insbesondere bei PCR-Verfahren mit gleichzeitiger Replikation eines internen Standards, sich die gleichzeitig ablaufenden Replikations-Reaktionen nicht gegenseitig beeinflussen, machen sich bei der erfindungsgemäßen parallel ablaufenden Konkurrenzreaktion die Nachweis- und die Kompetitor-Nucleinsäure zumindest einen der Bestandteile des Replikations-Reagens streitig. Dieses Phänomen wird auch Abfang- oder Kompetitiv-Reaktion genannt. Dementsprechend sind die Replikations-Effizienzen von Nachweis- und Kompetitor-Nucleinsäure voneinander abhängig: Je höher die Konzentration der einen Nucleinsäure ist, desto größer ist der Anteil am limitierenden Bestandteil, der zur Replikation dieser Nucleinsäure verwendet wird, und desto größer ist die Replikations-Effizienz dieser Nucleinsäure. Die Replikations-Effizienz der jeweils anderen Nudeinsäure ist entsprechend geringer, da ihr Anteil am limitierenden Bestandteil, der zur Replikation dieser anderen Nucleinsäure verwendet wird, geringer ist. Im Ergebnis unterdrückt die Replikation der Kompetitor-Nucleinsäure die Replikation insbesondere unspezifisch gebundener Nachweis-Nucleinsäure und senkt somit das Hintergrundsignal. Sie fördert die Replikation der Nachweis-Nucleinsäure nicht, sondern behindert diese sogar.

Die Kompetitor-Nucleinsäure umfasst in bevorzugten Ausführungsformen des erfindungsgemäßen Verfahrens Abschnitte, die mit Abschnitten der Nachweis-Nucleinsäure insoweit funktionell übereinstimmen, dass sie während der Replikation der Nachweis- und der Kompetitor-Nucleinsäure mit einer einzelnen Oligonucleinsäure des Replikations-Reagens zur Replikation hybridisieren können. In diesem Fall konkurrieren die Nachweis- und Kompetitor-Nucleinsäure bei ihrer Replikation um die betreffende Oligonucleinsäure.

Ferner ist es bevorzugt, wenn die Nachweis- und Kompetitor-Nucleinsäuren eine Länge von 50 bis 700 Nucleobasen besitzen. Nucleinsäuren mit solchen Längen lassen sich gut handhaben und verhältnismäßig leicht und sicher replizieren. Es hat sich herausgestellt, dass sich Primer- und Kompetitor-Nucleinsäuren mit einer Länge von 100 bis 300 Nucleobasen besonders gut replizieren und nachweisen lassen.

Dabei ist es bevorzugt, wenn sich die Nachweis- und die Kompetitor-Nucleinsäure lediglich in einer Erkennungssequenz von 15-40 Nucleobasen voneinander unterscheiden, wobei diese Unterschiede vorzugsweise in einem zusammenhängenden Bereich beabstandet von den Enden der Nachweis- und der Kompetitor-Nucleinsäure auftreten.

Vorzugsweise stimmt ein zusammenhängender Endabschnitt von zumindest 8, vorzugsweise von 10 bis 40 Nucleobasen Länge an einem jeweils entsprechenden Ende in Nachweis- und Kompetitor-Nucleinsäure mit höchstens zwei Abweichungen überein. Unter einer Abweichung wird dabei ein Basenaustausch, eine Deletion oder die Insertion einer Nucleobase verstanden. Besonders bevorzugt ist es, wenn dieser übereinstimmende Bereich am 5'-Ende jeweils der Nachweis- und der Kompetitor-Nucleinsäure beginnt (Primer-Bindungsstelle); ist die Nachweisund/oder die Kompetitor-Nucleinsäure keine DNA oder RNA, so beginnt der übereinstimmende Bereich vorzugsweise an dem Ende, das dem 5'-Ende eines einzelsträngigen linearen DNA-Strangs mit gleicher Nucleobasensequenz entspricht. Durch diese Maßnahmen wird erreicht, dass im Replikationsreagens möglichst wenige verschiedene Bestandteile vorhanden sein müssen, um sowohl die Nachweis- als auch die Kompetitor-Nucleinsäure replizieren zu können. Femer wird so erreicht, dass die Replikationseffizienz beider Nucleinsäuren ähnlich ist.

Bevorzugt sind solche Paare aus Nachweis- und Kompetitor-Nucleinsäure, die in einem Konzentrationsbereich von $10^{-20}$ bis $10^{-21}$ mol in Abwesenheit der jeweils anderen Nucleinsäure des Paares in etwa gleiche Replikationseffizienzen (Basis-Replikationseffizienzen) besitzen. Dies vereinfacht die Auswertung der Ergebnisse des erfindungsgemäßen Nachweisverfahrens, da keine unterschiedlichen Basis-Replikationseffizienzen berücksichtigt werden müssen.

Vorteilhafterweise liegt die Kompetitor-Nucleinsäure im Testansatz homogen gelöst vor.

Zur Replikation von Nucleinsäuren sind eine Reihe von Verfahren bekannt. Zu den bekannten Verfahren gehören insbesondere die Polymerase-Kettenreaktion (PCR), rolling-circle-Amplifikation (RCA), Ligase-Kettenreaktion (LCR) und nucleicacid-sequence-based-Amplifikation (NASBA). Diese ermöglichen es, auch kleinste Mengen einer Nucleinsäure bis hin zu wenigen hundert, zehn oder gar einem einzigen Molekül zu replizieren. Dadurch kann das Vorliegen eines einzigen oder weniger Moleküle der Nachweis-Nucleinsäure qualitativ und/oder quantitativ nachgewiesen werden. Eine bevorzugte Stellung nehmen dabei solche Replikationsverfahren ein, in deren Verlauf die Menge der replizierten Nachweis- und die Kompetitor-Nucleinsäure exponentiell zunimmt.

Replikations-Reagenzien sind Substanzen oder Substanzgemische zur Durchführung eines Replikationsverfahrens.

Zur Replikation der Nachweis- und der Kompetitor-Nucleinsäure und gegebenenfalls zu deren Nachweis sind insbesondere PCR-Verfahren geeignet und bevorzugt. Diese PCR-Verfahren werden wegen der Konkurrenz von Nachweis- und Kompetitor-Nucleinsäure um einen oder mehrere limitierende Bestandteile des Replikations-Reagens auch kompetitive PCR genannt. Die Durchführung von PCR-Verfahren ist seit langem gängige Praxis, so dass die zur Durchführung einzustellenden Verfahrensparameter anhand einfacher Vorversuche oder anhand des allgemeinen Fachwissens leicht optimiert werden können. Ferner sind die zur Durchführung solcher Verfahren benötigten Substanzen verhältnismäßig preiswert sowie leicht und schnell erhältlich. Die zur Durchführung der Replikation benötigten Geräte sind zudem in der überwiegenden Zahl der molekularbiologischen Forschungsinstitute und/oder Arbeitsgruppen bereits vorhanden.

Replikations-Reagenzien für die PCR umfassen üblicherweise eine Polymerase, eine Pufferlösung, Nucleotide wie desoxy-Adenosin-Triphosphat, desoxy-Cytidin-Triphosphat, desoxy-Guanosin-Triphosphat, desoxy-Thymidin-Triphosphat sowie einen oder mehrere Nucleinsäure-Primer als Startmoleküle für die Nukleinsäure-Replikation.

Besonders bevorzugt ist es dabei, wenn die Nachweis- und Kompetitor-Nucleinsäuren um die Primer eines PCR-Replikations-Reagens konkurrieren. Dazu sind üblicherweise die 5'-Endabschnitte der Nachweis- und der Kompetitor-Nucleinsäure wie oben beschrieben identisch oder ähnlich, wobei die Primer-Konzentration während der Replikation so niedrig gewählt wird, dass sie die maximale Replikations-Effizienz von Nachweis- und Kompetitor-Nucleinsäure limitiert. Zusätzlich oder alternativ dazu kann auch ein anderer Bestandteil des PCR-Replikations-Reagens für die Replikation der Nachweis- und der Kompetitor-Nucleinsäure limitierend sein. Dies kann insbesondere die Polymerase und/oder eines oder mehrere Nucleotide sein. In beiden Fällen können die Nucleobasen-Sequenzen der Nachweis- und der Kompetitor-Nucleinsäure voneinander verschieden sein; insbesondere kann ihre Homologie weniger als 80%, berechnet nach dem FASTA-Algorithmus von Pearson und Lipman (Improved tools for biological sequence comparison, *Proc. Natl. Acad. Sci. USA* 1988 Apr;85(8):2444-2448), betragen.

Der qualitative und vorzugsweise quantitative Nachweis der replizierten Nachweis-und der replizierten Kompetitor-Nucleinsäure kann, gegebenenfalls unter Einschalten eines Waschschrittes, auf zahlreiche verschiedene Weisen erfolgen. Insbesondere kann die replizierte Nachweis-Nucleinsäure und die replizierte Kompetitor-Nucleinsäure radioaktiv markiert sein/werden und (auto-)radiographisch nachgewiesen werden. Sie können jedoch auch mit Nucleinsäure-bindenden Farbstoffen, insbesondere Fluoreszenz-Farbstoffen, nachgewiesen werden. Dabei ist es besonders bevorzugt, wenn die Farbstoffe über eine zu einem Abschnitt der jeweiligen Nucleinsäure komplementäre Sonde an die jeweilige Nucleinsäure gebunden sind bzw werden. Die Sonde kann auch, wie in herkömmlichen Verfahren üblich, mit Enzymen verbunden sein, die selbst leicht nachweisbar sind oder die die Erzeugung eines leicht nachweisbaren Produkts bewirken. Alternativ können auch an die replizierten Nachweis- und Kompetitor-Nucleinsäuren selbst farbgebende Gruppen wie Fluoreszenz-Farbstoffe kovalent gebunden sein.

Bevorzugt ist eine Verfahrensgestaltung, bei der die replizierte Nachweis-Nucleinsäure und die replizierte Kompetitor-Nucleinsäure voneinander getrennt und unabhängig voneinander nachgewiesen werden. Eine solche Trennung kann auf vorteilhaft einfache Weise durch Gelelektrophorese der Nucleinsäuren selbst oder ggf. ihrer Abbauprodukte nach einem Verdau durch eine oder mehrere Restriktionsenzyme erfolgen.

Besonders bevorzugt ist es, die Nucleinsäuren durch Hybridisierungs-Verfahren auf der Basis von ELISA oder, besonders bevorzugt, ELOSA (U. Reischl, R. Rüger, C. Kessler, Nonradioactive labeling and high-sensitive detection of PCR products, *Mol. Biotechnol.* 1994, 229-240) zu trennen und nachzuweisen. Dabei werden die replizierte Nachweis- und die replizierte Kompetitor-Nucleinsäure durch Hybridisierung an spezifischen oberflächengebundenen Nucleinsäuren immobilisiert (ELOSA). Die Nucleinsäuren können dann anhand üblicher NachweisVerfahren nachgewiesen werden. Erste Versuche haben ergeben, dass es besonders vorteilhaft ist, die immobilisierten Nachweis- und Kompetitor-Nucleinsäuren mit Antikörpern oder anderen Bindungsmolekülen zu markieren, wobei die Antikörper Einheiten umfassen, die ein nachweisbares Signal erzeugen können. Solche Hybridisierungs-Verfahren ermöglichen eine vorteilhaft einfache gleichzeitige Durchführung mehrerer Nachweisverfahren. Dadurch wird auch die gleichzeitige Untersuchung einer größeren Zahl an Proben zum Nachweisen einer oder mehrerer nachzuweisender Substanzen ermöglicht. Die Nachweisgrenzen sind dabei häufig überraschend niedrig.

Das erfindungsgemäße Verfahren erlaubt es, preisgünstige Materialien zu seiner Durchführung zu verwenden. Insbesondere Nucleinsäuren, auch biotinylierte Nucleinsäuren, sind preiswert und schnell erhältlich. Ferner können die Nucleinsäuren weitgehend unabhängig vom Nachweis-Bindungsreagens und der nachzuweisenden Substanz ausgewählt werden. Dies erlaubt es, die im Verfahren verwendeten Nucleinsäuren aufeinander abgestimmt zu standardisieren.

Besonders bevorzugt ist dabei ein erfindungsgemäßes Verfahren, bei dem das Nachweiskonjugat

- zwei oder mehr miteinander verbundene Nachweis-Bindungsreagenzien umfasst und/oder
- zwei oder mehr Nachweis-Nucleinsäuren umfasst.

Damit ist ein Nachweis-Bindungsreagens im Nachweiskonjugat mit einem weiteren Nachweis-Bindungreagens und/oder den Nachweis-Nucleinsäuren verknüpft, und zwar nicht oder zumindest nicht nur über die nachzuweisende Substanzt. Das Nachweiskonjugat ist somit hinsichtlich des Nachweis-Bindungsreagens und/oder der Nachweis-Nucleinsäure oligomerisiert. Die erste Variante ermöglicht es auf vorteilhaft einfache Weise, die Beständigkeit der Bindung zwischen einem Nachweiskonjugat und einer nachzuweisenden Substanz zu erhöhen, da in einem solchen Konjugat mehrere Bindungsreagenzien in unmittelbarer Nähe zur nachzuweisenden Substanz vorliegen und an diese binden können. Die zweite Variante erhöht mit vorteilhaft einfachen Mitteln die Wahrscheinlichkeit, dass ein Nachweiskonjugat tatsächlich durch Replikation zumindest einer Nachweis-Nucleinsäure nachgewiesen wird.

Die beteiligten Reagenzien (Nachweis-Bindungsreagens, Nachweis-Nucleinsäure) können vorteilhafterweise auf gleiche Art verbunden sein bzw. werden wie zuvor beschrieben. Bevorzugt ist es, wenn die Nachweis-Bindungsreagenzien ein- oder mehrfach biotinyliert sind und die Nachweis-Nucleinsäuren ein- oder mehrfach biotinyliert oder so kovalent an Streptavidin gebunden sind, dass dessen Biotin-Bindungsfähigkeit nicht wesentlich herabgesetzt wird.

Vorteilhafterweise wird das Nachweisverfahren so durchgeführt, dass in einem weiteren Schritt e) die nachzuweisende Substanz an einer Oberfläche immobilisiert wird. Durch die Immobilisierung ist es auf vorteilhaft einfache Weise möglich, die nachzuweisende Substanz von anderen Substanzen abzutrennen bzw. sie an vorgewählten Orten zu konzentrieren.

Eine Oberfläche im Sinne eines solchen erfindungsgemäßen Verfahrens kann eine beliebige Oberfläche sein. Insbesondere kann es sich um eine Glas-, Silizium- oder Metalloberfläche oder eine andere, vorzugsweise zur Herstellung von Biochips geeignete Oberfläche handeln. Unter den Metalloberflächen sind Gold- oder goldbeschichtete Oberflächen bevorzugt. Es kann sich jedoch auch um Kunststoffoberflächen, insbesondere Polystyrol- oder Polycarbonat-Oberflächen handeln, wie sie insbesondere in Mikrotiterplatten-Kavitäten vorliegen. Bei der Oberfläche kann es sich auch um die Oberfläche metallischer Kolloide und vorzugsweise magnetischer Mikropartikel handeln, wie sie zur Extraktion von Nucleinsäuren aus Flüssigkeiten verwendet werden. Besonders bevorzugt sind Oberflächen, die mit einer Nucleinsäure verbunden werden können.

Die Oberfläche kann mit die Verfahrensdurchführung nicht störenden Substanzen beschichtet (geblockt) werden. Insbesondere kann die Oberfläche mit Gelatine, Milchpulver und/oder Rinderserum-Albumin (BSA) beschichtet sein. Die Oberfläche kann auch mit Nucleinsäuren versehen sein, soweit diese die Durchführung des Nachweisverfahrens nicht beeinträchtigen.

Besonders bevorzugt ist es, wenn zur Durchführung von Schritt e)

- die nachzuweisende Substanz an ein immobilisierungs-Bindungsreagens gebunden ist bzw. wird, das mit einer ersten Immobilisierungs-Nucleinsäure verbunden ist (Fängerkonjugat), und
- die erste Immobilisierungs-Nucleinsäure mit einer an einer Oberfläche immobilisierten zweiten Immobilisierungs-Nucleinsäure hybridisiert wird, um das Immobilisierungs-Bindungsreagens an der Oberfläche zu immobilisieren.

Dabei sind Fängerkonjugate Verbindungen aus zumindest einer ersten Immobilisierungs-Nucleinsäure und zumindest einem Immobilisierungs-Bindungsreagens zum Binden an die nachzuweisende Substanz.

Die beiden Immobilisierungs-Nucleinsäuren sind Nucleinsäuren der eingangs beschriebenen Art. Sie können also unabhängig voneinander und von den Nachweis-und Kompetitor-Nucleinsäuren DNA, RNA oder eines deren funktionalen Analoga (beispielsweise eine Peptidnucleinsäure) sein.

Das Immobilisierungs-Bindungsreagens kann aus der gleichen Gruppe ausgewählt werden wie das Nachweis-Bindungsreagens, wobei die mit der Auswahl verbundenen Vorteile ebenfalls übernommen werden können.

Die Bindungen von Immobilisierungs-Bindungsreagens und zweiter Immobilisierungs-Nucleinsäure können in Entsprechung zu den Bindungen von Nachweis-Bindungsreagens und Nachweis-Nucleinsäure ausgestaltet sein. Insbesondere ist es bevorzugt, wenn das Immobilisierungs-Bindungsreagens und die erste Immobilisierungs-Nucleinsäure durch Biotin-Streptavidin-Bindungen verbunden sind. Die zweite Immobilisierungs-Nucleinsäure kann auf herkömmliche Weise, beispielsweise über Linker-Moleküle, mit der Oberfläche verbunden sein.

In Entsprechung zu den Konjugaten aus Nachweis-Bindungsreagens und Nachweis-Nucleinsäure kann in einem Fängerkonjugat in Abwesenheit der nachzuweisenden Substanz ein Immobilisierungs-Bindungsreagens mit einem weiteren Immoblisierungs-Bindungreagens und/oder zwei oder mehreren ersten Immobilisierungs-Nucleinsäuren verknüpft (oligomerisiert) sein. Ebenfalls entsprechend ist in der ersten Variante die Stabilität der Bindung eines Fängerkonjugats an die nachzuweisende Substanz durch die Zahl der verfügbaren Bindungsreagenzien vorteilhaft erhöht, während in der zweiten Variante die Immobilisierung des Fängerkonjugats (gegebenenfalls einschließlich einer gebundenen nachzuweisenden Substanz) verbessert ist, indem mehrere erste und zweite Immobilisierungs-Nucleinsäuren zur Immobilisierung des Fängerkonjugats beitragen können.

Das Verfahren erlaubt es zudem, an die Oberfläche gebundene Fängerkonjugate wieder abzulösen und somit die Oberfläche zu regenerieren und die Fängerkonjugate, gegebenenfalls mit daran gebundener nachzuweisender Substanz, erneut in Lösung zu bringen.

Das Verfahren ermöglicht es außerdem, die Reihenfolge des Zusammenbringens von Fängerkonjugat, Nachweis-Bindungsreagenzien (ggf. mit daran gebundenen Nachweis-Nucleinsäuren) und nachzuweisender Substanz sowie der mit der zweiten Immobilisierungs-Nucleinsäure verbundenen Oberfläche weitgehend frei zu wählen und so an die nachzuweisende Substanz anzupassen, dass eine möglichst niedrige Nachweisgrenze erreicht wird.

Durch die Verwendung von Fängerkonjugaten kann zudem die Immobilisierung der nachzuweisenden Substanz an eine Oberfläche vorteilhaft lange hinausgezögert werden. Zudem können die Fängerkonjugate in Lösung, also ohne die Einschränkungen, denen sie bei einer herkömmlichen vorangehenden Immobilisierung unterliegen, an der nachzuweisenden Substanz binden. Beides ermöglicht eine Verbesserung der Nachweisgrenze.

Ein DNA-getriebenes Immobilisierungsverfahren (DNA-directed immobilization, DDI) ist bereits bekannt aus C.M. Niemeyer et al., *Analytical Biochemistry* 1999, 54-63. Dabei wird ein biotinyliertes Protein, insbesondere ein biotinyliertes Immunoglobulin, über Streptavidin mit einem ersten biotinylierten DNA-Einzelstrang zu einem Präkonjugat verknüpft. In einem weiteren Schritt wird an einer Oberfläche eine vorgewählte Menge einer zweiten einzelsträngigen Nucleinsäure angebracht, wobei der zweite DNA-Einzelstrang komplementär zum ersten Einzelstrang ist. Durch Hybridisierung von erstem und zweitem DNA-Einzelstrang wird das jeweils mit dem ersten DNA-Einzelstrang verknüpfte Protein an der Oberfläche immobilisiert.

Überraschenderweise hat sich nun herausgestellt, dass die Anwesenheit von DDI-Reagenzien (insbesondere Präkonjugate entsprechend der letztgenannten Veröffentlichung) die Durchführung des erfindungsgemäßen Nachweisverfahrens nicht behindert, sondern sogar zu einer Verbesserung, das heißt Erniedrigung, der Nachweisgrenze führen kann.

Bevorzugt ist ferner ein Verfahren, bei dem Schritt e) wie folgt durchgeführt wird:

- Zusammenbringen des Fängerkonjugats, des Nachweiskonjugats und der nachzuweisenden Substanz zum Herstellen eines Nachweis-Komplexes aus Fängerkonjugat, nachzuweisender Substanz und Nachweiskonjugat, und anschließend
- Zusammenbringen der mit der zweiten Immobilisierungs-Nucleinsäure verbundenen Oberfläche mit dem Fängerkonjugat, um das Fängerkonjugat durch Hybridisieren von erster und zweiter Immobilisierungs-Nucleinsäure an die Oberfläche zu binden.

Es hat sich nämlich herausgestellt, dass die Anlagerung eines Nachweiskonjugats an eine nachzuweisende Substanz im Einzelfall behindert sein kann, wenn diese bereits in immobilisierter Form vorliegt. Nach einem Vorab-Immobilisieren der nachzuweisenden Substanz würde zudem zweckmäßigerweise ein Waschschritt zum Entfernen nicht immobilisierter Stoffe durchgeführt, um die Spezifität des Nachweisverfahrens zu steigem. Beides führt aber zu einer Verschlechterung (Erhöhung) der Nachweisgrenze.

Es ist daher wünschenswert, ein erfindungsgemäßes Nachweisverfahren so zu gestalten, dass (a) die Immobilisierung der nachzuweisenden Substanz möglichst spät im Verfahren erfolgt oder (b) auf andere Weise bewirkt wird, dass die Immobilisierung der nachzuweisenden Substanz an eine Oberfläche die Bindung eines Nachweis-Reagens an die nachzuweisende Substanz wenig oder nicht behindert. Beides ist mit der bevorzugten Verfahrensgestaltung auf vorteilhaft einfache Weise möglich.

Insbesondere ist es bevorzugt, eine Lösung der nachzuweisenden Substanz mit einer Mischung aus Fänger- und Nachweiskonjugaten zu vereinigen, bevor die Fängerkonjugate über ihre erste Immobilisierungs-Nudeinsäure mit der an die Oberfläche gebundenen zweiten Immobilisierungs-Nudeinsäure hybridisiert werden. Auf diese Weise wird erreicht, dass ein hoher Anteil von Fänger- und Nachweiskönjugaten an die nachzuweisende Substanz binden kann, so dass die Nachweisgrenze vorteilhaft erniedrigt ist. Außerdem ist es auf diese Weise möglich, die nachzuweisende Substanz durch eine vorgefertigte Mischung von Fänger- und Nachweiskonjugaten in einen Nachweis-Komplex zu überführen. Für häufig nachzuweisende Substanzen lassen sich so die Anzahl der zur Durchführung des Verfahrens benötigten Lösungen minimieren sowie die Lösungen selbst vereinheitlichen und vorfabrizieren.

Bei der Herstellung der im erfindungsgemäßen Nachweisverfahren verwendeten Fänger- bzw. Nachweiskonjugate wird man so vorgehen, dass eine vorgewählte Menge des Nachweis- bzw. Immobilisierungs-Bindungsreagens und der jeweils zugehörende(n) Nucleinsäure(n) gemischt und auf geeignete Weise jeweils miteinander verbunden werden. Dabei wird am Ende ein Anteil der jeweiligen Bindungsreagenzien nicht mit jeweils zugehöriger Nucleinsäure verbunden sein.

Es ist nun ein Verfahren bevorzugt, bei dem das Fänger- und/oder das Nachweiskonjugat vor Durchführung der Schritte e) und/oder a) nicht von solchen Immobilisierungs- bzw. Nachweis-Bindungsreagenzien abgetrennt wird, die nicht mit dem Fänger- bzw. Nachweiskonjugat verbunden sind. Entgegen der Erwartung, dass nicht mit Fänger- bzw. Nachweiskonjugat verbundene Bindungsreagenzien um die Bindungsstellen auf der nachzuweisenden Substanz mit den Fänger- bzw. Nachweiskonjugaten konkurrieren und die Nachweisgrenze somit unvorteilhaft erhöhen, hat sich herausgestellt, dass auf die Abtrennung verzichtet werden kann, ohne die Nachweisgrenze wesentlich zu beeinträchtigen. Ein solches erfindungsgemäßes Verfahren ist daher in einer vorteilhaft niedrigen Anzahl von Arbeitsschritten und dementsprechend schnell und einfach durchführbar. Es vereinfacht daneben auch die Herstellung der Fänger- und/oder Nachweiskonjugaten.

Es ist natürlich noch immer möglich, das Fänger- und/oder das Nachweiskonjugat vor Durchführung der Schritte e) und/oder a) aufzureinigen. Auf diese Weise können ungebundene Bindungsreagenzien wiedergewonnen werden. Femer ermöglicht eine Aufreinigung die Abtrennung der Fänger- und/oder Nachweiskonjugate von anderen, im weiteren Verfahrenablauf gegebenenfalls störenden Substanzen.

Wenn die nachzuweisende Substanz mehrere Bindungsstellen zum Binden der Bindungsreagenzien besitzt oder sich mehrere nachzuweisende Substanzen verbinden, so dass der Verbund mehrere Bindungsstellen zum Binden der Bindungsreagenzien besitzt, so ist ein Verfahren besonders bevorzugt, bei dem das Nachweis- und das Immobilisierungs-Bindungsreagens im wesentlichen gleich oder identisch sind. Dabei sind zwei Bindungsreagenzien im wesentlichen gleich, wenn sie an gleichen oder einander räumlich überlappenden Stellen an der nachzuweisenden Substanz binden. Dies ist insbesondere der Fall, wenn zumindest die Bindungsreagens-Regionen, die für die Erkennung der nachzuweisenden Substanz verantwortlich sind, identisch oder einander sehr ähnlich sind.

Obwohl zu erwarten war, dass das Nachweis- und das Immobilisierungs-Bindungsreagens einander jeweils durch Konkurrenz um die Bindungsstellen auf der nachzuweisenden Substanz verdrängen und somit die Nachweisgrenze erhöhen würden, wurde überraschend gefunden, dass diese Konkurrenz keinen nachteiligen Effekt auf die mit dem erfindungsgemäßen Verfahren erzielbare Nachweisgrenze hat. Dementsprechend ist ein solches Verfahren vorteilhaft einfach durchführbar, da lediglich eine einzige Bindungsreagens-Sorte statt deren zwei zur Herstellung sowohl des Fänger- als auch des Nachweiskonjugats benötigt wird. Dies ermöglicht es insbesondere, einen monoclonalen Antikörper und/oder dessen Fragmente als Nachweis- und Immobilisierungs-Bindungsreagens zu verwenden. Dadurch wird die verhältnismäßig aufwendige Gewinnung eines zweiten monoclonalen Antikörpers umgangen.

Ferner ist ein Nachweisverfahren bevorzugt, bei dem die Menge der replizierten Nachweis- und Kompetitor-Nucleinsäuren bestimmt wird. Dies kann, in Abhängigkeit vom gewählten Nachweisverfahren der Nucleinsäuren, beispielsweise durch Messung der Intensität einer durch die Nucleinsäuren hervorgerufenen Färbung, Schwärzung eines Röntgenfilms oder auf andere Weise geschehen.

Besonders bevorzugt ist dabei ein Verfahren, bei dem zusätzlich zu einem Testansatz in einem weiteren Ansatz (Negativkontrolle) ohne Gegenwart der nachzuweisenden Substanz, jedoch unter vorheriger identischer Durchführung sämtlicher beteiligten Bindungs- und Waschschritte

f) das Replikations-Reagens unter den Bedingungen behandelt wird, wie sie für eine Nucleinsäure-Replikation der Nachweis- und der Kompetitor-Nucleinsäuren benötigt werden,

g) die Menge der in der Negativkontrolle gegebenenfalls replizierten Nucleinsäuren bestimmt wird und

h) die Mengen replizierter Nachweis- und Kompetitor-Nucleinsäuren mit der Menge der in der Negativkontrolle gebildeten Nucleinsäuren verglichen werden.

In der Negativkontrolle liegen somit mit Ausnahme der nachzuweisenden Substanz alle Stoffe in den Konzentrationen vor, wie sie auch im Testansatz vorliegen. In der Negativkontrolle liegt insbesondere die Kompetitor-Nucleinsäure in der gleichen Konzentration vor wie im Testansatz. Die nachzuweisende Substanz kann in der Negativkontrolle durch ein ihr gleiches Volumen an Wasser, einem Puffer oder einer Probe einer biologischen Matrix ohne die nachzuweisende Substanz ersetzt werden. Negativkontrolle und Testansatz werden ansonsten gleich behandelt, insbesondere wird vor Durchführung der Replikation der Nucleinsäuren in einem Waschschritt solche Nachweis-Nucleinsäure zumindest weitgehend entfernt, die nicht in einem Nachweis-Komplex an nachzuweisende Substanz gebunden ist.

Idealerweise unterscheiden sich Negativkontrolle und Testansatz lediglich darin, dass die Negativkontrolle keine nachzuweisende Substanz enthält. Dies ist jedoch nicht immer möglich, insbesondere, wenn die nachzuweisende Substanz in einer komplexen Probe wie Blutserum oder eine andere biologische Matrix vorliegt. In diesem Fall ist es bevorzugt, wenn sich die Negativkontrolle - außer in der An- und Abwesenheit der nachzuweisenden Substanz - vom Testansatz so wenig wie möglich unterscheidet. Dies kann beispielsweise bei Blutserum-Proben dadurch erreicht werden, dass anstelle des die nachzuweisende Substanz enthaltenden Serums eine gleich große Menge standardisierten Serums (bei Humanserum-Proben beispielsweise "BISEKO" der Firma Biotest) in der Negativkontrolle verwendet wird.

Ebenfalls bevorzugt ist ein Verfahren bei dem als Negativkontrollle eine authentische Probe der biologischen Matrix verwendet wird, die möglicherweise die nachzuweisende Substanz enthalten könnte, in der die nachzuweisende Substanz jedoch durch Zugabe von Bindungsreagenzien "maskiert" wird. Eine solche Maskierung kann beispielsweise durch Zugabe von Bindungsmolekülen in einer geringen, an vergleichbaren gespikten Proben durch equilibrieren ermittelten Menge erfolgen, die eine Bindung der nachzuweisenden Substanz an die verfahrensgemäßen Bindemoleküle im Nachweiskonjugat verhindern, durch ihre geringe Menge jedoch die Matrixeigenschaften nicht oder nur vernachlässigbar gering beeinflussen. Durch eine solche Maskierung lässt sich in experimentell einfacher Weise eine Negativkontrolle aus dem eigentlichen Probenmaterial generieren. Dieses Vorgehen ist insbesondere dann vorteilhaft, wenn keine Negativkontrollen verfügbar sind, beispielsweise wenn Hirnhomogenate, Blutproben oder andere Matrices auf das Vorhandensein von Prionproteinen untersucht werden sollen. Da es in solchen Fällen oftmals zum Auftreten Individuum-spezifischer Nullwerte, sogenannte "Hintergrundwerte" durch unspezifische Bindung von Matrixbestandteilen kommt, ist das Vorhandensein einer Negativkontrolle vorteilhaft hilfreich bei der Festlegung von Schwellenwerten beispielsweise für die Analyse von Prionproteinen im Him individueller Tiere. Hier kann nun ein Aliquot der zu untersuchenden Matrixprobe maskiert werden und steht damit als Negativkontrolle zur Verfügung.

Alternativ kann als Negativkontrolle auch partiell aufgereinigte Matrixprobe eingesetzt werden, in der das nachzuweisende Substanz durch spezielle Methoden entfernt wurde. Beispielsweise kann die Entfernung der nachzuweisenden Substanz durch Affinitätsreinigungsschritte mit Hilfe spezieller Bindungsreagenzien oder durch geeignete Extraktionsverfahren erfolgen.

Die Menge der in der Negativkontrolle replizierten Nucleinsäuren kann durch eines der zuvor beschriebenen Verfahren bestimmt werden.

Zum Vergleich der Mengen replizierter Nachweis- und Kompetitor-Nucleinsäuren mit der Menge der in der Negativkontrolle gebildeten Nucleinsäuren ist es bevorzugt, das Verhältnis der replizierten Mengen an Nachweis- und Kompetitor-Nucleinsäure durch das Verhältnis der Mengen der in der Negativkontrolle replizierten Nachweis- und Kompetitor-Nucleinsäuren zu dividieren. Sei also $N_T$ ein Maß für die mittlere Menge der replizierten Nachweis-Nucleinsäure aus einem Testansatz, $K_T$ ein Maß für die mittlere Menge der replizierten Kompetitor-Nucleinsäure aus dem Testansatz und $N_N$ ein Maß für die mittlere Menge der replizierten Nachweis-Nucleinsäure aus der Negativkontrolle, $K_N$ ein Maß für die mittlere Menge der replizierten Kompetitor-Nucleinsäure aus der Negativkontrolle, so gilt:

$$Q = (N_T / K_T) / (N_N / K_N)$$

worin Q der Vergleichsquotient ist. $N_T$, $K_T$, $N_N$ und $K_N$ können insbesondere die Mittelwerte von Doppel- oder Dreifachbestimmungen von ELOSA-Messungen der jeweiligen Nucleinsäure-Mengen sein. Es ist dabei bevorzugt, die Größen $N_N$ und $K_N$ genauer zu bestimmen als die Größen $N_T$ und $K_T$. Sind letztere beispielsweise die Mittelwerte aus Doppelbestimmungen, so sind $N_N$ und $K_N$ Mittelwerte zumindest aus Dreifachbestimmungen. Auf die Verwendung absoluter Einheiten (insbesondere SI-Einheiten) für diese Größen ($N_T$, $K_T$, $N_N$ und $K_N$) kann verzichtet werden; stattdessen können auch relative Einheiten wie beispielsweise eine Farbintensität oder die Schwärzung eines Röntgenfilms als Maß für die Menge einer Nucleinsäure verwendet werden.

Der Vergleichsquotient kann als Maß für den Erfolg der Replikationsreaktion dienen. Ist der Vergleichsquotient Q zu niedrig, beispielsweise kleiner als 1,5 zu 1, so zeigt dies, dass die Konzentration der nachzuweisenden Substanz im Testansatz unterhalb der Nachweisgrenze lag. Die Replikation der Nucleinsäuren im Testansatz lieferte dann im wesentlichen das gleiche Ergebnis wie die Replikation der Nudeinsäuren in der Negativkontrolle.

Je größer der Wert des Vergleichsquotienten Q, desto höher war die Ausgangskonzentration der Nachweis-Nucleinsäure im Testansatz. Sind die Ausgangskonzentration der Kompetitor-Nucleinsäure in Testansatz und Negativkontrolle bekannt, so kann die Ausgangskonzentration der Nachweis-Nucleinsäure berechnet werden.

Zweckmäßig ist es dabei, zunächst in einem Kalibrierungsschritt die nachzuweisende Substanz in mehreren verschiedenen vorgewählten Konzentrationen $c_1$ - $c_n$ nachzuweisen und die jeweiligen Vergleichsquotienten $Q_1$ - $Q_n$ zu bestimmen. Dies entspricht einer "externen Standardisierung" die es ermöglicht, unbekannte Mengen an der nachzuweisenden Substanz zu quantifizieren. Die Vergleichsquotienten $Q_1$ - $Q_n$ werden gegen die vorgewählten Konzentrationen $c_1$ - $c_n$ aufgetragen. Die Parameter der durch mathematische Analyse ermittelten Funktion des Zusammenhangs zwischen dem Vergleichsquotienten und der Konzentration werden bestimmt. Anhand dieser Funktion kann bei Proben unbekannter Konzentration die Konzentration der nachzuweisenden Substanz bestimmt werden.

Bevorzugt ist es, durch Variation der Bedingungen des Nachweisverfahrens oder die Wahl des verwendeten Konzentrationsbereiches $c_1$ - $c_n$ eine lineare Abhängigkeit zwischen der Konzentration und dem Vergleichsquoteinten zu erhalten.

Während bei einer gewöhnlichen PCR das Verhältnis zwischen der Menge der replizierten Nucleinsäure und der Ausgangsmenge der zu replizierenden Nucleinsäure erst bei hohen Nucleinsäure-Konzentrationen linear ist, wird durch die Verwendung der Kompetitor-Nucleinsäure überraschend bewirkt, dass der lineare Zusammenhang zwischen den Mengen an nachzuweisender Substanz und replizierten Nachweis- und Kompetitor-Nucleinsäuren bereits bei niedrigen Konzentrationen hergestellt wird. Dies vereinfacht die Auswertung der Messergebnisse. Möglicherweise spielt die Kupplungseffizienz zwischen nachzuweisender Substanz und der Nachweis-Nucleinsäure bei diesem Linearisierungs-Effekt eine wichtige Rolle.

Ein mathematischer Zusammenhang zwischen dem Vergleichsquotienten Q und der Konzentration oder Menge der nachzuweisenden Substanz ist eine notwendige Voraussetzung für das erfindungsgemäße Quantifizierungsverfahren unter Verwendung einer Negativkontrolle, ein linearer Zusammenhang gestaltet diese Quantifizierung vorteilhaft einfach.

Durch die Verwendung des Vergleichsquotinenten Q als Kontrollsignal kann auf vorteilhaft einfache Weise erreicht werden, dass sich das Kontrollsignal auch bei geringen Ausgangskonzentrationen an Nachweis-Nucleinsäuren im Testansatz deutlich von zufälligen Werten, wie sie beispielsweise durch nicht völlig abgewaschene Nachweiskonjugate in der Negativkontrolle verursacht werden, unterscheidet. Daher wird durch den Vergleichsquotienten Q eine Sensitivitätssteigerung des Nachweisverfahrens erzielt (eine Definition des Begriffs Sensitivität ist zu finden in C. P. Price, D. J. Newman, *Principle and Practice of Immunoassay*, Macmillan Reference Ltd. 1997: 180ff). Versuche haben ergeben, dass das erfindungsgemäße Nachweisverfahren umso höhere Sensitivitäts-Steigerungen im Vergleich zu herkömmlichen Nachweisverfahren wie ELISA und umso geringere Fehlerbreiten liefert, je niedriger die Konzentration der nachzuweisenden Substanz und je komplexer die Probenmatrix ist. In solchen Fällen können so niedrige Nachweisgrenzen erreicht werden, dass selbst der Nachweis und die Quantifizierung homöopathisch verabreichter Wirkstoff-Dosierungen möglich ist.

Femer ist ein erfindungsgemäßes Verfahren bevorzugt, bei dem die nachzuweisende Substanz bei Durchführung des erfindungsgemäßen Nachweisverfahrens in einer biologischen Matrix vorliegt. Dabei sind biologische Matrices Substanzen oder Substanzgemische, die in biologischen Proben enthalten sind. Insbesondere sind Seren. Blutserum, Körperflüssigkeiten wie Urin, Speichel, Sperma, Rückenmarksflüssigkeit, Hirnlysate, Gewebehomogenate und Aufschlüsse aus biologischem Material wie Zell- oder Gewebeaufschlüsse biologische Matrices, ebenso wie die Bestandteile der aufgezählten Substanzen und Gemische. Das erfindungsgemäße Verfahren erlaubt es vorteilhafterweise, die nachzuweisende Substanz vor Durchführung des Nachweisverfahrens nicht oder nicht vollständig von solchen biologischen Matrices abtrennen zu müssen. Dadurch wird das Nachweisverfahren vereinfacht und die bei einer Aufreinigung auftretenden Verluste an nachzuweisender Substanz werden begrenzt. Die durch den Kompetitor zusätzlich erhöhte Sensitivität im Vergleich zur bekannten Immuno-PCR Methode, die ohne Kompetitor durchgeführt wird, vereinfacht ebenfalls die Arbeit in biologischen Matrices. Die Sensitivität von Nachweisverfahren ist bei Verwendung biologischer Matrices gewöhnlich herabgesetzt gegenüber der Sensitivität von Nachweisverfahren, bei denen die nachzuweisende Substanz in einem reinen Puffer vorliegt. Bei Verwendung des erfindungsgemäßen Nachweisverfahrens tritt diese Schwierigkeit jedoch nicht oder nur in geringerem Umfang auf.

Ferner ist ein erfindungsgemäßes Verfahren bevorzugt, bei dem die nachzuweisende Substanz in wässriger Lösung Aggregate bildet. Aggregatbildenden Substanzen sind insbesondere lipophile und amphiphile Substanzen. Die Aggregate können in herkömmlichen Nachweisverfahren ausfallen oder diese auf sonstige Weise beeinträchtigen. Insbesondere führt die Aggregatbildung dazu, dass ein Anteil der im günstigsten Fall verfügbaren Bindungsreagens-Bindungsstellen auf der nachzuweisenden Substanz im Inneren der Aggregate verborgen (maskiert) ist und zur Bindung der Bindungsreagenzien tatsächlich nicht zur Verfügung steht. Es hat sich jedoch überraschend herausgestellt, dass die Nachweisgrenze eines erfindungsgemäßen Verfahrens für solche Substanzen vorteilhaft niedriger liegt als zu erwarten war. Dies gilt insbesondere für den Fall, dass als Nachweis- und/oder Immobilisierungs-Bindungsreagens ein Immunglobulin, insbesondere ein IgG-Antikörper, oder eines der eingangs beschriebenen Fragmente eines Immunglobulins verwendet wird.

Besonders bevorzugt ist ein Verfahren, bei dem die nachzuweisende Substanz ein Prionprotein ist. Das Prionprotein (PrP) gilt als ein wichtiges, wenn nicht gar als das auslösende übertragbare Agens neurodegenerativer Krankheiten wie Scrapie, Creutzfeldt-Jakob-Krankheit, bovine spongiforme Encephalopathie und vergleichbarer Krankheiten. Ein Übersichtsartikel von J. Collinge ist in *Hum.Mol.Genet.* 1997, 1699 bis 1705, erschienen. Der Ausdruck "prion protein" wurde von S.B. Prusiner, *Science* 1982, 136 bis 144, zur Kennzeichnung des infektiösen Agens der Scrapie-Krankheit verwendet und hat sich seitdem zum Inbegriff einer ganzen Klasse wirkungsmäßig vergleichbarer Proteine entwickelt (siehe auch Bodemer, *Naturwissenschaften* 86, 212). Solche Substanzen neigen in wässriger Lösung besonders stark zur Bildung von Aggregaten, die die Bindung von Immunglobulinen (Antikörpern) in herkömmlichen Verfahren stark einschränken und somit nur in unvorteilhaft hoher Konzentration nachweisbar sind. Es hat sich jedoch überraschend herausgestellt, dass der Nachweis von Prionproteinen in komplexen Matrices wie Himhomogenaten oder Blutserumproben mit einem erfindungsgemäßen Nachweisverfahren auch bei niedrigen Konzentrationen des Prionproteins möglich ist.

Ebenfalls bevorzugt ist ein Verfahren, bei dem die nachzuweisende Substanz ein Lektin, vorzugsweise ein Mistel-Lektin ist. Auch Lektine wie Mistel-Lektine neigen in wässriger Lösung stark zur Bildung von Aggregaten, die die Bindung von Immunglobulinen (Antikörpern) in herkömmlichen Verfahren stark einschränken und somit nur in unvorteilhaft hoher Konzentration nachweisbar sind. Es hat sich jedoch überraschend herausgestellt, dass der Nachweis von Lektinen, insbesondere von Mistel-Lektinen, mit einem erfindungsgemäßen Nachweisverfahren auch bei niedrigen Konzentrationen des Lektins möglich ist.

Dabei werden unter Prion-Proteinen und Mistel-Lektinen auch solche Substanzen verstanden, die hervorgegangen sind aus der Expression rekombinanter Prionoder Lektin-Gene Insbesondere sind rViscumin (siehe beispielsweise Schmidt A, Mockel B, Eck J, Langer M, Gauert M, Zinke H: *Cytotoxic activity of recombinant bFGF-rViscumin fusion proteins*, Biochem Biophys Res Commun 2000 Oct 22;277(2);499-506 sowie Eck, J., M. Langer, B. Mockel, A. Baur, M. Rothe, H. Zinke und H. Lentzen (1999), "Cloning of the mistletoe lectin gene and characterization of the recombinant A-chain." *Eur J Biochem* **264**(3): 775-84 sowie Eck, J., M. Langer, B. Mockel, K. Witthohn, H. Zinke und H. Lentzen (1999), "Characterization of recombinant and plant-derived mistletoe lectin and their B-chains. " *Eur J Biochem* **265**(2): 788-97) und kommerziell erhältliches rekombinantes PrPc (Prionics AG Recombinant bovine PrP Product No. 03-010) Mistel-Lektine bzw. Prion-Proteine im Sinne des erfindungsgemäßen Nachweisverfahrens

Es ist zudem in einer Vielzahl von Fällen ein Verfahren bevorzugt, bei dem die Schritte a) und gegebenenfalls e) im wesentlichen frei von Detergenzien durchgeführt werden. Detergenzien, also chaotrope Stoffe wie beispielsweise Guanidiniumhydrochlorid und grenzflächenaktive Stoffe und Tenside wie beispielsweise TWEEN-20 und TWEEN-80, werden in herkömmlichen Verfahren verwendet, um in wässrigen Lösungen Aggregate lipophiler oder amphiphiler Substanzen zu spalten. Dabei bedeutet im wesentlichen frei von Detergenzien, dass die Konzentration von Detergenzien (grenzflächenaktiven Stoffen und Tensiden) geringer ist als 1 Vol.-%. Vorzugsweise ist die Konzentration sogar geringer als 0,2 Vol.-%, besonders bevorzugt geringer als 0,1 Vol.-%. Die Verwendung von Detergenzien stört häufig die Bindung von Bindungsreagenzien an die nachzuweisende Substanz und wird deshalb in herkömmlichen Verfahren nach Möglichkeit vermieden. Bei stark aggregierenden Substanzen, insbesondere lipophilen oder amphiphilen Substanzen, wird jedoch die Verwendung von Detergenzien als unumgänglich angesehen. Es hat sich nun überraschenderweise herausgestellt, dass bei Verwendung erfindungsgemäßer Fänger- und/oder Nachweis-Komplexe die Menge an benötigten Detergenzien im Vergleich zu herkömmlichen Verfahren erheblich gesenkt werden kann oder dass sogar auf die Verwendung von Detergenzien völlig verzichtet werden kann. Dies ermöglicht es, eine überraschend niedrige Nachweisgrenze zu erzielen. Hinzu kommt, dass die Durchführung des Nachweisverfahrens durch den Verzicht auf Detergenzien vorteilhaft vereinfacht wird.

Ferner ist ein Kit zur Durchführung eines erfindungsgemäßen Nachweisverfahrens vorgesehen, umfassend

a) ein Nachweis-Bindungsreagens und gegebenenfalls ein Immobilisierungs-Bindungsreagens,
b) eine Nachweis- und gegebenenfalls eine Immobilisierungs-Nucleinsäure,
c) Mittel zum Verbinden des Nachweis-Bindungsreages mit der Nachweis-Nucleinsäure, und gegebenenfalls Mittel zum Verbinden des Immobilisierungs-Bindungsreagens mit der Immobilisierungs-Nucleinsäure,
d) eine Kompetitor-Nucleinsäure.

Mit einer Zusammenstellung von Substanzen in einem solchen erfindungsgemäßen Kit können ein Nachweis- und gegebenenfalls Fängerkonjugate der beschriebenen Art vorteilhaft einfach hergestellt werden. Zudem wird die Durchführung eines erfindungsgemäßen Verfahrens durch das Vorsehen einer Kompetitor-Nucleinsäure in der gleichen Packungseinheit wie die Nachweis-Nucleinsäure erleichtert.

Die Erfindung wird nachfolgend anhand der Figuren und einiger Ausführungsbeispiele näher beschrieben. Es stellen dar:

Fig 1 Schema des hochsensitiven Nachweises von Substanzen mittels Bindungsmolekülen und Nucleinsäure-Amplifikation

Fig. 2 Schema des Einsatzes von Nachweis- und Kompetitor-Nucleinsäure

Fig. 3 Schema der Datenerzeugung und -prozessierung

Fig. 4 Nachweis von rViscumin in Blutserumproben

Figur 1 zeigt schematisch den hochsensitiven Nachweis einer Substanz durch Kupplung mit einer Nachweis-Nucleinsäure und anschließender enzymatischer Amplifizierung dieser Nucleinsäure. Die nachzuweisende Substanz (Kreis) wird in einem Sandwich-Assay unter Verwendung eines ersten Bindungsmoleküls (1, Y-förmig dargestellt) aus einer biologischen Matrix (kleine Quadrate) isoliert, während ein zweites Bindungsmolekül (2, Y-förmig dargestellt) genutzt wird, die nachzuweisende Substanz mit einer Nachweis-Nucleinsäure (3) zu kuppeln. Nach Entfernung unspezifisch gebundener Reagenzien durch einen Waschschritt erlaubt eine anschließende Nucleinsäure-Amplifikation (PCR) die exponentielle Vervielfältigung der Nachweis-Nudeinsäure und über die abschließende Detektion der amplifizierten Nucleinsäure (4) die Quantifizierung der nachzuweisenden Substanz. Dieses Verfahren ist aufgrund der Leistungsfähigkeit der Nucleinsäure-Amplifikation deulich sensitiver als ein vergleichbares ELISA-Verfahren, in welchem die nachzuweisende Substanz mit einem Bindungsmolekül-Enzymkonjugat (5) gekuppelt wird.

Figur 2 zeigt schematisch den Ablauf des Nachweises unter Aufbau eines Nachweiskomplexes (E) aus einer nachzuweisenden Substanz, beispielsweise einem Antigen (C), einem Nachweis-Reagens-Oligomer (A), einem Fänger-Reagens-Oligomer (B) und einer Oberfläche, die spezifisch das Fänger-Reagens-Oligomer bindet (D). Dabei enthält das Nachweis-Reagens-Oligomer Antigen-spezifische Bindungsmoleküle (Y-förmig dargestellt) sowie eine Nachweis-Nucleinsäure (Doppelpfeil). Das Fänger-Reagens-Oligomer ist aus Antigen-spezifischen Bindungsmolekülen (Y-förmig dargestellt) und Nucleinsäuren (Pfeil) aufgebaut, die spezifisch an oberflächengebundene komplementäre Nucleinsäuren (entgegengesetzter Pfeil) binden können. Der die Nachweis-Nucleinsäure enthaltende immobilisierte Nachweiskomplex (E) wird in Gegenwart einer definierten Menge einer Kompetitor-Nucleinsäure (F), die sich von der Nachweis-Nucleinsäure durch die Gegenwart einer spezifischen Erkennungssequenz (weisses Rechteck) unterscheidet, einer PCR-Nucleinsäureamplifikation unterzogen. Wenn ursprünglich eine grosse Menge Antigen (Kreis) vorliegt, wurde eine grosse Menge Nachweis-Nucleinsäure immobilisiert und Fall G tritt ein: Im PCR-Produkt wird eine grosse Menge des Amplifikates der Nachweis-Nucleinsäure und nur eine geringe Menge des Amplifikates der Kompetitor-Nucleinsäure gefunden. Wenn hingegen ursprünglich eine geringe Menge Antigen vorgelegen hat, tritt Fall H ein: Im PCR-Produkt wird nur eine geringe Menge des Amplifikates der Nachweis-Nucleinsäure und eine grosse Menge des Amplifikates der Kompetitor-Nucleinsäure gefunden.

Zum getrennten Nachweis der PCR-Amplifikate von Nachweis- und Kompetitor-Nucleinsäure werden diese beispielsweise zunächst im PCR-Schritt mit einem Hapten (Fünfeck) markiert. Das markierte Amplifikatgemisch (I) wird im Anschluß an die PCR thermisch denaturiert und die markeirtem Einzelstränge (J) durch ortsaufgelöste Hybridiserung an komplementäre Oligonucleotide, welche entweder spezifisch die Nachweis-Nucleinsäure oder die Erkennungssequenz der Kompetitor-Nucleinsäure binden, voneinander getrennt. Die abschließende Detektion kann dann beispielsweise über ein Antikörper-Enzymkonjugat gegen das in die Nucleinsäuren eingefügte Hapten erfolgen.

Figur 3 beinhaltet eine schematische Wiedergabe der Datenerzeugung und - prozessierung im erfindungsgemäßen Verfahren an einem Beispiel:

a.) Zu untersuchen ist eine Reihe verschiedener Proben "T", bezeichnet als "1", "2" und "3" die die nachzuweisende Substanz enthalten (repräsentiert durch schwarze Quadrate) sowie eine Negativkontrolle "N" ohne die nachzuweisende Substanz, bezeichnet mit "NC" (repräsentiert durch ein weisses Quadrat).
b.) Diese Proben werden zunächst in einer Doppelbestimmung in unterschiedlichen Kavitäten eines Mikrotiter-Moduls immobilisiert und mit Nachweis-Nucleinsäure gekuppelt.

c.) Nach Amplifikation in Gegenwart einer Kompetitor-Nucleinsäure werden pro Probe zwei Amplifikationsprodukte erhalten: Das Amplifikat der Nachweis-Nucleinsäure (leere Quadrate) und der Kompetitor-Nucleinsäure (mit "IC" gekennzeichnete Quadrate)

d.) Diese Amplifikate wurden im Anschluss beispielsweise mit einem PCR-ELOSA in Doppelbestimmung nachgewiesen, so dass pro Probe bzw. Negativkontrolle ein Set von acht Messwerten, je vier für die Kompetitor- und die Nachweis-Nucleinsäure erhalten werden.

e.) Zur Auswertung wird zunächst aus den je vier Messwerten je Probe "T" für Nachweis- und Kompetitor-Nucleinsäure der Mittelwert "$N_T$" bzw. "$K_T$ sowie der Fehler der Methodik ermittelt (exemplarisch gezeigt an Probe 1)

f.) Aus den Mittelwerten der für Nachweis- und Kompetitor-Nucleinsäure erhaltenen Signalintensitäten wird im Anschluß der Quotient $N_T$ / $K_T$ gebildet.

g.) Somit wird für jede Probe und die Negativkontrolle ein Quotientenwert, symbolisiert durch einen Kreis, berechnet

h.) Die Quotientenwerte $N_T$ / $K_T$ der die nachzuweisende Substanz enthaltenden Proben (1,2,3) werden jeweils durch den Quotientenwert $N_N$ / $K_N$ der Negativkontrolle (NC) dividiert.

i.) Als abschließendes Ergebnis (symbolisiert durch Dreiecke) werden als "Vergleichsquotienten" Q die auf die Negativkontrolle normierte Werte erhalten, die den Gehalt der nachzuweisenden Substanz jeweils als Vielfaches der Negativkontrolle wiedergeben. Dieses Signal-zu-Hintergrund-Verhältnis der Vergleichs-Quotientenwerte ist dabei bessser als eine bloße Normierung der Signalintensitäten einer Nachweis-Nucleinsäure einer Probe ohne Genewart der Kompetitor-Nucleinsäure auf die Signale der Nachweis-Nucleinsäure einer Negativkontrolle ohne nachzuweisenden Substanz.

Figur 4 zeigt die Ergebnisse der Quantifizierung von rViscumin in BISEKO, einem standardisierten kommerziell erhältlichen Humanserum (siehe 7. Ausführungsbeispiel). Kurve 1 in Abbildung A zeigt die in einem ELOSA bestimmten Mittelwerte der Signalintensitäten des Amplifikates der Nachweis-Nucleinsäure ($N_T$), Kurve 2 in Abbildung A die Mittelwerte der Signalintensitäten des Amplifikates der Kompetitor-Nucleinsäure ($K_T$). Kurve 1 in Abbildung B gibt die auf die Negativkontrolle normierten Werte eines ELISA-Nachweisverfahrens für rViscumin wieder, Kurve 2 und 3 in Abbildung B die normierten Werte eines Nachweisverfahrens mit (3) und ohne (2) Einbeziehung der Kompetitor-Nucleinsäure "KF-1". Zum Erhalt der normierten Werte in Gegenwart der Kompetitor-Nucleinsäure wurde entsprechend der in Fig. 3 gezeigten Vorgehensweise zunächst der Quotient $N_T$ / $K_T$ aus den Signalen für Nachweis- und Kompetitor-Nucleinsäure gebildet, bevor zum Erhalt des Vergleichs-Quotienten (Q) auf die Negativkontrolle normiert wurde.

**1. Ausführungsbeispiel: Synthese und Test eines Paares aus Nachweis-und Kompetitornucleinsäure mit identischen Primerbindungsstellen**

In diesem Ausführungsbeispiel wurde artifiziell zu einer Nachweis-Nucleinsäure mit besonders günstigen Eigenschaften eine Kompetitor-Nucleinsäure mit identischen Primerbindungsstellen, annähernd gleicher Länge und einer spezifischen Erkennungssequenz hergestellt.

1.1. Auswahl der Nachweis-Nucleinsäure

Zum Design der Basensequenzen für die in der kompetitive Nucleinsäure-Amplifizierung verwendeten Nachweis- und Kompetitor-Fragmente werden zunächst verschiedene dopplesträngige endständig 5'-biotinylierte NucleinsäureFragmente hinsichtlich ihrer Eignung als Nachweis-Nucleinsäure in einem hochsensitiven Proteinassay untersucht. Hierzu wurde nach bekanntem Protokoll (C.M. Niemeyer et al., Nucleic Acids Res. 1999, 4553 bis 4561) der Nachweis von Maus-IgG mit verschiedenen DNA-Fragmenten des kommerziell erhältlichen Plasmides M13mp18 (New England Biolabs) durchgeführt. Ein geeignetes Fragment wird dadurch charakterisiert, dass es etwa 0,01 attomol Maus-IgG nachzuweisen vermag (Siehe Tabelle 1).

Tabelle 1:

| Eignung verschiedener DNA-Fragmente für die Immuno-PCR von Maus-IgG. Verwendet wurden die DNA-Fragmente DNA-42 (Position 6210-6251 auf M13mp18), DNA-104 (Position 6252-6364 auf M13mp18) und DNA-169 (Position 6252-6354 auf M13mp18) Angegeben sind jeweils normierte Signalintensitäten bezogen auf die gleich 1 gesetzte Negativkontrolle ohne Antigen. Die besten Sensitivitäten wurden dabei mit dem DNA-Fragment DNA-169 erzielt. | | | |
|---|---|---|---|
| **Maus-IgG [amol]** | **DNA-42** | **DNA-104** | **DNA-169** |
| **1** | 1,5 | 1,5 | 3 |
| **0,1** | 1 | 1,3 | 2,5 |
| **0,01** | 1 | 1 | 1,3 |
| **Negativkontrolle ohne Maus- IgG** | 1 | 1 | 1 |

Die doppeisträngigen Nachweis-Fragmente wurden durch quantitative PCR amplifiziert, dabei nach bekannten Verfahren endständig mit Biotinylsubstituenten derivatisiert und nach ebenfalls bekannten Verfahren zur Synthese eines oligomeren Streptavidin-Konjugats genutzt (C.M. Niemeyer et al., Nucleic Acids Res. 1999, 4553 bis 4561). Aufgrund der hohen Sensitivität beim Nachweis des Modell-Antigens wurde DNA-169 als Nachweis-Nucleinsäure ausgewählt (Stand der Technik).

1.2. Auswahl der Erkennungssequenzen für die Nachweis- und Kompetitor-Nucleinsäure

Im Anschluß wurde eine spezifische Fänger-Nucleinsäure so ausgewählt, dass sie eine hohe Signalintensität im Zuge einer Festphasen-Hybridisierung des Nachweis-Fragments liefert. Dabei erlaubte es die Verwendung von DNA-169 als Nachweis -Nucleinsäure auf bekannte Untersuchungen über die Hybridiserungseigenschaften einzelner etwa 20 Basenpaar langer Abschnitte der Nucleinsäure zurückzugreifen werden (Niemeyer, C. M., L. Boldt, et al. (1999). "Evaluation of single-stranded nucleic acids as carriers in the DNA- directed assembly of macromolecules." J Biomol Struct Dyn 17(3): 527-38). In diesen Untersuchungen zeigte die Sequenz "N-Es" (siehe Anhang) optimale Hybrisisierungseigenschaften. Daher wurde sie als Erkennungssequenz der Nachweis-Nucleinsäure ausgewählt (Stand der Technik).

Als Erkennungssequenz für die Kompetitor-Nucleinsäure wurde die künstliche Sequenz "K-Es" ausgewählt, die in Hybridiserungsexperimenten nach der in der Literatur beschriebenen Vorgehensweise (Niemeyer, C. M. et al. (1999), J Biomol Struct Dyn 17(3): 527-38) eine der Sequenz "B" sehr ähnliche Hybridisierungseffizienz aufwies (siehe Tabelle 2).

Tabelle 2:

| Vergleich der Hybridisierungseffizienzen der Erkennungssequenzen der Nachweis-Nucleinsäure ("N-Es") und der Kompetitor-Nucleinsäure ("K-Es"). Der Nachweis erfolgte jeweils über einen ELOSA unter Verwendung der komplementären Fänger-Oligonucleotide. Angegeben sind realtive Hybridisierungseffizienzen, dabei wurde das für Sequenz "N-Es" erhaltene Signal gleich 100% gesetzt. Es wurde nur eine vemachlässigbar geringe unspezifische Bindung der Erkennungssequenzen an das jeweils für die andere Erkennungssequenz spezifische Fänger-Oligonucleotid beobachtet. | | |
|---|---|---|
| **Sequenz** | **cB** | **31** |
| **Hybridisierungseffizienz an das zu Sequenz "N-Es" komplementäre Oligonucleotid** | 100% | <1 % |
| **Hybridisierungseffizlenz an das zu Sequenz "K-Es" komplementäre Oligonucleotid** | <1 % | 108% |

1.3. Synthese der Kompetitor-Nudeinsäure ..KF-1"

Auf Basis des Plasmids pUC-19 (New England Biolabs) wurde artifiziell eine Kompetitor-Nucleinsäure zum Nachweis-Fragment DNA-169 hergestellt, das so wohl die Primerbindugsstellen "N-1" und "N-2" (alle Primer: Interactiva, Sequenzen siehe Anhang) des Nachweisfragmentes als auch die Erkennungssequenz "K-Es" enthält. Zu diesem Zweck wurde eine "Anker"-PCR (C.R. Newton, A. Graham, "PCR" (Labor im Focus), Spektrum Akademischer Verlag, 2. Auflage 1994) verwendet. Bei dieser Vorgehensweise wurden mittels der speziellen Primer "K-1" und "K-2" (Sequenzen siehe Anhang) an die Enden eines 117bp DNA-Fragmentes des Plasmides pUC-19 die ausgewählte Erkennungssequenz "K-Es" sowie die benötigten Primerbindungsstellen des Nachweisfragmentes angefügt. Als Produkt einer präparativen PCR mit diesen beiden Primern und dem pUC-19 Plasmid als Template wird das "KF-1" Fragment (Sequenz siehe Anhang) amplifiziert. Nach Ablauf der PCR wurde der Reaktionsansatz in einem 2,5 %igen Agarose-Gel elektrophoretisch aufgetrennt, die Produkt-Bande nach Ethidiumbromid-Färbung aus dem Gel eluiert. Mit dem Eluat als Template wurde dann unter Verwendung der Primer der Nachweis-Nudeinsäure eine präparative PCR zur Synthese einer Stammlösung der Kompetitor-Nucleinsäure "KF-1" durchgeführt. Die Durchführung der präparativen PCR sowie die anschließende Aufreinigung des Amplifikate erfolgen dabei nach bekannten Verfahren (C.M. Niemeyer et al., Nucleic Acids Res. 1999, 4553 bis 4561)

1.4. Test der Kompetitor-Nucleinsäure "KF-1"

Zum Test der kompetitiven co-Amplifizierbarkeit der Nachweis- und Kompetitor-Nucleinsäure wurde eine konstante Menge von $10^{-19}$ Mol je PCR-Ansatz des Kompetitor-Fragmentes "KF-1" mit unterscheidlichen Mangen der Nachweisnucleinsäure vermischt und in Gegenwart von Digoxigenin-dUTP (Roche) einer PCR mit den Primern "N-1" und "N-2" unter Standard-Parametern (siehe C.M. Niemeyer et al., Nucleic Acids Res. 1999, 4553 bis 4561) mit einer Annealing-Temperatur von 55°C unterzogen. Der Nachweis der Amplifikate erfolgte analog eines in der Literatur beschriebenen PCR-ELOSAs (Niemeyer, C. M. et al. (1999), J Biomol Struct Dyn 17(3): 527-38) unter Verwendung eines Anti-Digoxigenin-Alkalische-Phosphatase-Konjugates (Roche). Nachweis- und Kompetitor-Nucleinsäure wurden dabei durch Hybridisierung ihrer spezifischen Erkennugssequenzen "N-Es" und "K-Es" an die als Fänger-Oligonucleotide auf einer Streptavidin-beschichteten Oberfläche immobilisierten komplementären biotinylierten Nucleinsäuren "cN-Es" und "cK-Es" (Interactiva) getrennt, Dieses Vor gehen ermöglicht den empfindlichen, quantifizierbaren und reproduzierbaren enzymatischen Nachweis der Amplifikate. Dabei wurde für eine maximale Sensitivität des Assays das Fluoreszenz-generierenden Substrat AttoPhos™ (Roche) der alkalischen Phosphatase eingesetzt und mittels eines VICTOR 1420 Multilabelcounters (Wallac) das Fluoreszenzsignal vermessen.

1.5. Ergebnisse:

Es wurde mit der Nachweis-Nucleinsäure "DNA-169" und der Kompetitor-Nucleinsäure "KF-1" ein Paar co-amplifizierbarer Nucleinsäuren, die jeweils eine spezifische Erkennungssequenz mit ähnlichen Hybridisierungseigenschaften aufweisen. Durch die Verwendung identischer Primer und die Wahl geeigneter Konzentrationsverhältnisse wird eine kompetitive Co-Amplifikation von Markerund Nachweisnucleinsäure beobachtet (siehe Tabelle 3).

| | $1x10^{-18}$ | $1x10^{-19}$ | $1x10^{-20}$ | $1x10^{-21}$ | $1x10^{-22}$ | mol "DNA-169" |
|---|---|---|---|---|---|---|
| **A,.) Signal "DNA-169"** | 450 | 300 | 110 | 20 | 2 | |
| **B.) Signal "KF-1"** | 150 | 410 | 590 | 600 | 610 | |
| **C.) Signal „DNA-169" ohne Gegenwart von „KF-1"** | | 300 | 120 | 90 | 10 | |

Tabelle 3: Kompetetive Co-Amplifikation von Nachweis-Nukleinsäure „DNA-169" und Kompetitor-Nucleinsäure „KF-1". In Gegenwart einer konstanten Menge von $1x10^{-19}$ mol „KF-1" wurde eine PCR unterschiedlicher ;Mengen der „DNA-169" durchgeführt. Dabei erweist sich die Höhe der in einer nachfolgenden PCR-ELOSA bestimmten Signalintensität für das Amplifikat der Kompetitor-Nucleinsäure „KF-1" (B.) in einem Bereich bis zu etwa $1x10^{-22}$ mol „DNA-169" abhängig von der Menge der Nachweis-Nucleinsäure. Angegeben sind jeweils relative Signalintensitäten bezogen auf eine gleich Eins gesetzte PCR-Negativkontrolle ohne Nucleinsäure-Templat. Vergleicht man die für die Nachweis-Nucleinsäure „DNA-169" erhaltenen Signalintensitäten in Gegenwart der Kompetitor-Nucleinsäure „KF-1" (A.) mit denen einer PCR ohne Gegenwart des Kompetitors (C.), so wird eine Reduzierung der Signalintensität insbesondere bei geringen Mengen der Nachweis-Nucleinsäure deutlich.

**2. Ausführungsbelspiel: Verwendung eines Paares aus Nachweis- und Kompetitor-Nucleinsäure mit unterschiedlichen Primer-bindungsstellen zum Nachweis des Modell-Antigens Kaninchen-IgG**

In diesem Ausführungsbeispiel wird das aufgrund seiner co-Amplifizierbarkeit mit der Nachweis-Nucleinsäure "DNA-169" ausgewählte DMA-Fragment "KF-2" zum hochsensitiven Nachweis des Modell-Antigens Kaninchen-IgG eingesetzt. Diese Kompetitor-Nucleinsäure unterscheidet sich mit 30 bp Differenz sowohl deutlich in ihrer Länge als auch in den Primerbindungsstellen von der Nachweis-Nucleinsäure "DNA-169".

2.1. Test der kompetitiven co-Amplifizierbarkeit von "DNA-169" und "KF-2"

Zu einer variablen Menge der Nachweis-Nucleinsäure "DNA-169" (siehe erstes Ausführungsbeispiel) von $1\times10^{-17}$ bis $1\times10^{-23}$ mol pro PCR-Ansatz wurde eine konstante Menge von $1\times10^{-20}$ mol der Kompetitor-Nucleinsäure "KF-2" (Sequenz siehe Anhang) pro PCR-Ansatz gegeben. Unter Verwendung der Primer "N-1", "N-2", "H-1" und "H-2" (alle Primer: Interactiva, Sequenzen siehe Anhang) wurde ein PCR unter den bekannten Standardbedingungen (C. M. Niemeyer et al., Nucleic Acids Res. 1999, 4553 bis 4561) mit einer Annealingtemperatur von 55°C durchgeführt. Darüber hinaus wurden Kontrollexperimente mit unterschiedlichen Mengen zwischen $1\times10^{-23}$ und $1\times10^{-17}$ mol pro PCR-Ansatz der Kompetitor-Nucleinsäure "KF-2" durchgeführt.

2.2. Nachweis von Kaninchen-IgG

Das bekannte Standardprotokoll eines hochsensitiven Kaninchen-IgG Nachweises mittels Antikörper-DNA-Aggregaten (C.M. Niemeyer et al., Nucleic Acids Res. 1999. 4553 bis 4561) wurde um die Zugabe der Kompetitor-Nucleinsäure "KF-2" in einer Menge von $1\times10^{-20}$ mol pro PCR-Ansatz ergänzt und die anschließende PCR-Amplifikation ebenfalls in Gegenwart der 4 Primer "N-1", "N-2", "H-1" und "H-2" durchgeführt. Zur Kontrolle wurde der Nachweis ebenfalls ohne den Kompetitor "KF-2" durchgeführt.

2.3. Nachweis der PCR-Amplifikate von "DNA-169" und "KF-2"

Der Nachweis der Amplifikate erfolgte analog eines in der Literatur beschriebenen PCR-ELOSAs (Niemeyer, C. M. et al. (1999), J Biomol Struct Dyn 17(3): 527-38) unter Verwendung der 5'-biotinylierten Fänger-Oligonucleotidsequenzen "H-1" und "cN-Es" sowie eines Anti-Digoxigenin-Alkalische-Phosphatase-Konjugates (Roche). Zusätzlich wurde wie in der Literatur beschrieben (Niemeyer, C. M., M. Adler und D. Blohm (1997), "Fluorometric Polymerase Chain Reaction (PCR) Enzyme-Linked Immunosorbent Assay for Quantification of Immuno-PCR Products in Microplates." *Anal Biochem* **246**(1); 140-5) eine Gel-Elektrophorese des PCR-Amplifikats in einem 2% nicht-denaturierenden Agarose-Gel durchgeführt, um die Amplifikate von Nachweis- und Kompetitor-Nucleinsäure anhand ihrer Länge zu unterscheiden. Dabei wurde die Graustufen-Intensität der Banden als Meßwert aufgenommen.

2.4. Ergebnisse:

Die Banden der Amplifikationsprodukte der Nachweis-Nucleinsäure "DNA-169" und der Kompetitor-Nucleinsäure "KF-2" lassen sich deutlich in der Gelektrophorese voneinander unterscheiden.

Bei Zugabe einer konstanten Menge von $1\times10^{-20}$ mol pro PCR-Ansatz der Kompetitor-Nucleinsäure "KF-2" wurde eine kompetetive Amplifikation mit der Nachweis-Nucleinsäure "DNA-169" in einem Bereich von $1\times10^{-19}$ bis $1\times10^{-23}$ mol "DNA-169" beobachtet. Höhere Mengen an "DNA-169" unterdrücken die Bildung von Amplifikaten der Kompetitor-Nucleinsäure "KF-2". In Gegenwart von mehr als $1\times10^{-19}$ mol der Kompetitor-Nucleinsäure "KF-2" vor der PCR sind keine Amplifikate der Nachweis-Nucleinsäure "DNA-169" in Mengen unterhalb $1\times10^{-21}$ mol DNA vor der PCR detektierbar. Eine coAmplifikation der beiden Nucleinsäuren ist also nur in einem bestimmten Konzentrationsfenster von Nachweis- und Kompetitor-Nucleinsäure möglich.

Zum ;Nachweis des Kaninchen-IgGs wurde zur Auswertung zunächst der Quotient der erhaltenen Signalinsitäten des Amplifikats von Nachweis- und Kompeti tor-Nucleinsäure gebildet. Dies wurde sowohl für die Antigen enthaltenden Proben als auch für eine Negativkontrolle ohne Antigen durchgeführt. Im Anschluß daran wurde der Vergleichsquotient aus diesen Werten gebildet und mit dem Signal-zu-Hintergrundwert der Nachweis-Nucleinsäure "DNA-169" in Gegenwart der Kompetitor-Nucleinsäure "KF-2" sowie ohne Gegenwart der Kompetitor-Nucleinsäure verglichen.

a.) PCR-ELOSA

| | |
|---|---|
| **Zu quantifizierender Testansatz, enthaltend 0,1 attomol Kaninchen-IgG:** | |
| $O_T$: Sighalintensität der Nachweis-Nucleinsäure im PCR-ELOSA (ohne Gegenwart von "KF-2" in der PCR) | 4487 a.u. |
| $N_T$:Signalintensität der Nachweis-Nucleinsäure im PCR-ELOSA: ($1\times10^{-20}$ mol "KF-2" in der PCR) | 1525 a.u. |
| $K_T$: Signalintensität der Kompetitor-Nucleinsäure im PCR-ELOSA: ($1\times10^{-20}$ mol "KF-2" in der PCR) | 1163 a.u. |
| **Negativkontrolle ohne Kaninchen-IgG:** | |
| $O_N$: Signalintensität der Nachweis-Nucleinsäure im PCR-ELOSA (ohne Gegenwart von "KF-2" in der PCR) | 4114 a.u. |
| $N_N$:Signalintensität der Nachweis-Nucleinsäure im PCR-ELOSA: ($1\times10^{-20}$ mol "KF-2" in der PCR) | 1324 a.u. |
| $K_N$: Signalintensität der Kompetitor-Nucleinsäure im PCR-ELOSA: ($1\times10^{-20}$ mol "KF-2" in der PCR) | 1569 a.u. |
| Signal-zu-Hintergrundwert $S = O_T / O_N$ ohne "KF-2" | 1,1 |
| Signal-zu-Hintergrundwert $S = N_T / N_N$ in Gegenwart von "KF-2" | 1,2 |
| Vergleichsquctient $Q = (N_T / K_T) / (N_N / K_N)$: | 1,6 |

Der Vergleichsquotient Q, erhalten durch die Bildung der Teilquotienten aus den erhaltenen ELOSA-Werten für Nachweis- und Kompetitor-DNA, ergibt mit 1,6 einen deutlich höheren Wert für den 0,1 attomol Kaninchen- IgG enthaltenden Testansatz als die normalerweise übliche Bestimmung des Signal-zu-Hintergrundwertes S.

Ohne Gegenwart der Kompetitor-Nucleinsäure wurde unter identischen Bedingungen lediglich ein Signal-zu-Hintergrundwert von 1,1 für 0,1 attomol Kaninchen-IgG erhalten. Mit einem herkömmlichen ELISA ist unter vergleichbaren Bedingungen kein Kaninchen-IgG unterhalb einer Menge von 10 attomol mehr nachweisbar.

b.) Gel-Elektrophorese

| | |
|---|---|
| Signal-zu-Hintergrundwert S = $O_T$ / $O_N$ ohne "KF-2" | 1,4 |
| Signal-zu-Hintergrundwert S = $N_T$ / $N_N$ in Gegenwart von "KP-2" | 1,6 |
| Vergleichsquotient Q = ($N_T$ / $K_T$) / ($N_N$ / $K_N$): | 3 |

Der Vergleichsquotient Q, erhalten durch die Bildung der Teilquotienten aus den erhaltenen Bandenintensitäts-Werten für Nachweis- und Kompetitor-DNA, ergibt mit 3 einen deutlich höheren Wert für den 1 attomol enthaltenden Testansatz als die normalerweise übliche Bestimmung des Signal-zu-Hintergrundwertes S.

Ohne Gegenwart der Kompetitor-Nucleinsäure wurde unter identischen Bedingungen ledifglich ein Signal-zu-Hintergrundwert von 1,1 für 1 attomol Kaninchen-IgG erhalten. Mit einem herkömmlichen ELISA ist unter vergleichbaren Bedingungen kein Kaninchen-IgG unterhalb einer Menge von 10 attomol mehr nachweisbar.

**3. Ausführungsbeispiel: Nachweis von rekombinantem PrPc in Himhomogenat mittels eines Fänger- und eines Nachweis-Reagenzes unter Einsatz der Competitor-Nucleinsäure "KF-1"**

In diesem Anwendungsbeispiel wurden Fänger- und Nachweis-Reagens-Oligomere zum Nachweis von PrPc, der nicht-Krankheits-assoziierten Form des Prion-Proteins, eingesetzt. In einem konventionellen Assay der Krankheitsassoziierten Form des Prion-Proteins, $PrP_{sc}$, wird zunächst ein Proteinase K Abbau des PrPc durchgeführt, um im Anschluß mit Antikörpern, die sowohl $PrP_{sc}$ als auch PrPc erkennen, einen Nachweis des Proteinase K resistenten Antigens PrPSC zu ermöglichen. Als Modellsystem für diese Vorgehensweise wurden Proteinase-K verdaute Proben von bovinem Hirngewebe-Homogenat mit definierten Konzentrationen an PrPc versetzt. Der Nachweis wurde mittels der kombinierten Inkubation der PrPc enthaltenden Proben, Fänger-Reagens-Oligomer und Nachweis-Reagens-Oligomer auf einer mit einer ersten Fänger-Nucleinsäure beschichteten Oberfläche und anschließender PCR zur Amplifikation der im Nachweis-Reagens-Oligomer enthaltenen dritten Nucleinsäure in Gegenwart der Kompetitor-Nucleinsäure "KF-1" durchgeführt. Die abschließende Detektion der PCR-Amplifikate erfolgte durch einen PCR-ELOSA.

3.1. Beschichten von Mikrotiter-Modulen mit der ersten Nucleinsäure

Die nicht-kovalente Verknüpfung der Reaktionsgefäße mit der zur Immobilisierung des Fänger-Reagens-Oligomers benötigten ersten Nucleinsäure erfolgte durch Physisorption des Kupplungsproteins Streptavidin (STV) an die zu funktionalisierende Oberfläche und anschließender Inkubation mit biotinylierter Nucleinsäure (Niemeyer, C. M., L. Boldt, B. Ceyhan und D. Blohm (1999), "DNA-Directed immobilization: efficient, reversible, and site-selective surface binding of proteins by means of covalent DNA-streptavidin conjugates." *Anal Biochem* **268**(1): 54-63).

3.1.1.Immobilisierung des STV

Hierzu wurde zunächst eine 0,2 μM Lösung von rekombinantem core-STV (IBA, Göttingen) in PBS-Puffer (200 mM Phosphat ($KH_2PO_4$ / $K_2HPO_4$), pH 7) hergestelit. Je Kavität wurden 30 μl dieser Lösung auf Top-Yield Mikrotitermodule (Nunc) aufgetragen, für eine Minute bei 4000 upm in einer Zentrifuge mit Mikrotiterplattenrotor zentrifugiert, und für 72 h bei 4°C inkubiert. Nicht gebundenes STV wurde im Anschluss durch dreimaliges, je dreiminütiges Waschen mit 220 μl TBS-Puffer (20 mM Tris-Cl, 150 mM NaCl, pH 7,5) je Kavität entfernt. Danach wurde für mindestens 12h bei 4°C ein Blockierungsschritt gegen unspezifische Wechselwirkungen mit 240 μl MESTBS-Puffer (TBS, 45% Milchpulver (Oxoid), 0,2% $NaN_3$, 5 mM EDTA, 1 mg/ml DNA MB-grade (Roche)) pro Kavität durchgefuhrt. Die STV-beschichteten Mikrotitermodule können bis zu eine Woche mit diesem Puffer gelagert werden.

3.1.2. Kupplung mit biotinylierter Nucleinsäure

Zur Immobilisierung des Fänger-Reagens-Oligomers, welches die Nucleinsäure-Sequenz "A" (Sequenz siehe Anhang) enthält, wurde die komplementäre biotinylierte Nucleinsäure "bcA" (Sequenz siehe Anhang) verwendet. Eine 100 µM Stammlösung von bcA in TE-Puffer (10 mM Tris-Cl, 1mM EDTA, pH 7,5), gelagert bei -20°C, wurde unmittelbar vor Gebrauch in TETBS-Puffer (TBS, 5 mM EDTA, 0,05% Tween-20) auf eine Konzentration von 0,24 µM verdünnt.

Die STV-beschichteten und geblockten Mikrotitermodule wurden je zweifach für eine Minute und zweifach für fünf Minuten mit 220 µl TETBS pro Kavität gewaschen (im folgenden als: "Standard-Waschschritt" bezeichnet), und für 30 Minuten mit 30 µl je Kavität der verdünnten Lösung der biotinylierten Nucleinsaure bcA inkubiert.

Abschließend wurde dreifach für jeweils drei Minuten mit 220 µl je Kavität TETBS-Biotin (TETBS, 800 µM Biotin) gewaschen, um die verbleibende Biotinbindungsfähigkeit der STV-beschichteten Module abzusättigen. Die solcherart vorbereiteten Mikrotitermodule sind für 12 h bei 4°C mit 100 µl je Kavität TETBS-Biotin lagerbar.

3.2. Herstellung des Fänger-Reagens-Oligomers

3.2.1 Verwendeter biotinylierter Antikörper

Als Bindungsmoleküle zur Synthese von Nachweis- sowie Fänger-Reagens-Oligomeren stehen zur Zeit eine Reihe möglicher Antikörper zur Verfügung, die zum Teil bereits kommerziell erhältlich sind (z.B. 6H4, 5369, 6664, siehe Tabelle 5).

Im folgenden Anwendungsbeispiel wurde der kommerziell erhältliche Antikörper 6H4 (Prionics. Produkt-Nr. 01-010) verwendet.

Für die Kupplung des Antikörpers mit den Nucleinsäuren des Nachweis- sowie des Fänger-Reagens-Oligomers wurde das Biotin/STV-System verwendet (Diamandis and Christopoulos 1991). Der kommerziell nur ohne Biotinylierung erhältliche Antikörper wurde daher nach einem Standardverfahren mit Biotin gekuppelt (Meier, T. und F. Fahrenholz (1996), "A laboratory Guide to Biotin-Labeling in Biomolecule Analysis." *BioMethods 7* (Birkhäuser Verlag, Basel), 9-13): Zu einem Moläquivalent Antikörper erfolgte die Zugabe von 10 Moläquivalenten Sulfo-NHS-Biotin (Sigma) und anschließende Reinigung über NAP-10-Sephardex-Säulen (Pharmacia).

Der so erhaltene biotinylierte Antikörper wurde für die Synthese des Nachweissowie des Fänger-Reagens verwendet.

Tabelle 5:

| Antikörper zum Nachweis von PrP | | | |
|---|---|---|---|
| **Antikörper** | **Typ** | **Spezies** | **Quelle** |
| **6H4** | monoclonal | Maus | Prionics |
| **34C9** | monoclonal | Maus | Prionics |
| **3F4** | monoclonal | Maus | veröffentlicht von Kascsak, R. J., R. Rubenstein, P. A. Merz, M. Tonna-DeMasi, R. Fersko, R. I. Carp, H. M. Wisniewski und H. Diringer (1987), "Mouse polyclonal and monoclonal antibody to scrapie-associated fibril proteins." *J Virol* **61**(12): 3688-93 |
| **5369** | polyclonal | Ziege | Biogenesis Ltd. |
| **6664** | polyclonal | Ziege | Abcam |

3.2.2. Kovalentes Aggregat aus Streptavidin und zweiter Nucleinsäure

Zur Kupplung des biotinylierten Antikörpers mit der Nucleinsäure-Sequenz "A" (siehe 3.1.) wurde zunächst ein kovalentes Konjugat "HA" aus STV und einer thiolierten Nucleinsäure "A" hergestellt. Die Synthese dieses Konjugats unter Verwendung eines heterobifunktionellen chemischen Crosslinkers ist in der Literatur ausführlich beschrieben (Niemeyer, C. M., T. Sano, C. L. Smith und C. R. Cantor (1994), "Oligonucleotide-directed self-assembly of proteins: semisynthetic DNA-streptavidin hybrid molecules as connectors for the generation of macroscopic arrays and the construction of supramolecular bioconjugates." *Nucleic Acids Res* **22**(25): 5530-9). Kurzgefasst wurde zunächst in ein kommerziell erhältliches 5'-Amino-modifiziertes Oligonucleotid (Interactiva) eine Thiol-Gruppe eingeführt und diese im Folgeschritt mittels Sulfo-SMCC (Pierce) mit einer Maleimid-aktivierten primären Amino-Gruppe des STV verknüpft. Das nach anschließender Ionenaustausch-Chromatographie-Reinigung auf einer FPLC (Pharmacia) erhaltene gereinigte Produkt HA kann gewöhnlich bei 4°C mehrere Monate in TE gelagert werden.

3.2.3. Kupplung des Nachweis-Antikörpers mit der zweiten Nucleinsäure

Zur Herstellung des Fänger-Reagens-Oligomers wurden vier Picomol des Konjugates HA mit einer äquimolaren Menge des biotinylierten Antikörpers in einem Volumen von 8 µl TE-Puffer für 20 min bei Raumtemperatur unter orbitalem Schütteln inkubiert.

Ein Mikroliter dieser Lösung wurde im Anschluss in 500 µl RDB (9 Teile TETBS + 1 Teil MESTBS) aufgenommen. Durch Zugabe eines Mikroliters TETBS-Biotin und Inkubation für 5 min bei Raumtemperatur unter orbitalem Schütteln wurden die verbleibenden Biotinbindungsstellen des Fänger-Reagens-Oligomers abgesättigt. Die Herstellung des Fänger-Reagens-Oligomers erfolgte erst unmittelbar vor Verwendung, die erhaltene Lösung "FRO" kann für 30 min auf Eis gelagert werden. Die Lösung wurde nicht weiter gereinigt oder von unumgesetzten Edukten befreit.

3.3. Herstellung des Nachweis-Reagens-Oligomers

Für die Verknüpfung des biotinylierten Antikörpers mit der im PCR-Nachweis als Templat dienenden dritten Nucleinsäure wurde ein in der Literatur beschriebenes selbst-assembliertes Aggregat aus doppelt biotinylierter Nucleinsäure, tetravalentem Streptavidin und biotinyliertem Antikörper eingesetzt (Niemeyer, C. M., M. Adler, B. Pignataro, S. Lenhert, S. Gao, L. Chi, H. Fuchs und D. Blohm (1999), "Self-assembly of DNA-streptavidin nanostructures and their use as reagents in immuno-PCR." *Nucleic Acids Res* **27**(23): 4553-61). Als Nucleinsäure wurde dabei das bereits im ersten Ausführungsbeispiel beschriebene DNA-Fragment "DNA-169" verwendet.

Zur Herstellung des Nachweis-Reagens-Oligomers wurde in einem Volumen von 10 µl TE zu einer vorgelegten Menge von 2,5 pmol STV (IBA, siehe 3.1.) ein halbes Moläquivalent von 1,25 pmol der doppelt biotinylierten DNA "AG" gegeben und für 15 min bei Raumtemperatur unter orbitalem Schütteln inkubiert. Zwei Microliter der Lösung des STV-DNA-Aggregats wurden im Anschluss mit 0,25 pmol biotinyliertem Antikörper in einem Volumen von 5 µl TE für 20 min bei Raumtemperatur unter orbitalem Schütteln inkubiert. Zwei Microliter dieser Lösung des Antikörper-STV-DNA-Aggregats wurden in 500 µl RDB aufgenommen. Durch Zugabe eines Mikroliters TETBS-Biotin und Inkubation für 5 min bei Raumtemperatur unter orbitalem Schütteln wurden die verbleibenden Biotinbindungsstellen des Nachweis-Reagens-Oligomers abgesättigt. Die Herstellung des Nachweis-Reagens-Oligomers erfolgt erst unmittelbar vor Verwendung, die so erhaltene Lösung "DRO-1" ("Detektionsreagens-Oligomer") kann für 30 min auf Eis gelagert werden und wurde nicht weiter gereinigt oder von unumgesetzten Edukten befreit.

3.4. Kupplung mit PrPc

Als Antigen wurde in diesem Anwendungsbeispiel das kommerziell erhältliche bovine PrPc (Prionics, Produkt-Nr. 03-011) verwendet.

Als biologische Matrix wurden Himgewebe-Proben verwendet, die nach einer üblichen Standardvorgehensweise homogenisiert und mit Prorteinase K umgesetzt worden sind (vergleiche Martin, H.D., Blümel, C., Sandmöller, A., Kaiser, F., Kintrup, M., und Fetzer, J., BSE Aktuell. LaborPraxis, 2001. 25. Jahrgang, Nr. 2: p. 38-59).

Unter Verwendung dieser biologischen Matrix wurde eine Verdünnungsreihe PrPc mit Konzentrationen zwischen 10 ng/µl und 1 fg/µl PrPc sowie eine Negativkontrolle ohne PrPc in in silikonisierten 1,5 ml Reaktionsgefäßen (Biozym) vorbereitet.

Für die Kupplung des PrPc wurden zunächst jeweils 35 µl "FRO" und 35 µl "DRO-1" in silikonisierten 1,5 ml Reaktionsgefäßen (Biozym) vorgelegt. Zu diesem Gesamtvolumen von 70 µl wurden je 23,3 µl der PrPc-Lösungen in Himhomogenat gegeben und gut durchmischt. Der Ansatz wurde in einer Doppelbestimmung von 30 µl je Kavität sofort auf die Nucleotid-beschichteten Mikrotitermodule (siehe 3.1.) gegeben und für eine Stunde bei 37°C unter orbitalem Schütteln inkubiert.

3.5. Polymerase-Kettenreaktion (PCR)

Zum Nachweis der im Nachweis-Reagens-Oligomer enthaltenen dritten Nucleinsäure "DNA-169" wurde eine enzymatische Vervielfältigung mittels PCR durchgeführt (Niemeyer, Adler et al. 1999). Vor diesem Amplifikationsschritt wurde zunächst zweifach für je eine Minute und fünffach für je fünf Minuten mit 220 µl TETBS je Kavität unter orbitalem Schütteln bei Raumtemperatur gewaschen. Abschließend wurde noch zweifach für je eine Minute mit 220 µl TBS je Kavität gewaschen, um das PCR-hemmende EDTA des TETBS-Puffers aus den Kavitäten zu entfernen.

Die Module wurden mit Klebefolie (Plate Sealers, Dynex) verschlossen. Die Durchführung der PCR erfolgte in einem Gene Amplifying System 9600 (Perkin Eimer) oder alternativ in einem PTC-200 (MJ-Research) Thermalzykler mit 96-well Heizblock und aktiviertem Heizdeckel. Die PCR wurde wie im ersten Ausführungsbeispiel beschrieben durchgeführt.

Der PCR-Mastermix enthielt dabei 2 fM der Kompetitor-Nucleinsäure "KF-1", die Primer "N-1"und "N-2" sowie Digoxigenin-dUTP (Roche).

Der Nachweis der Amplifikate von Nachweis- und Kompetitor-Nucleinsäure erfolgte, wie bereits im ersten Ausführungsbeispiel beschrieben, mittels eines PCR-ELOSAs.

3.6. Kontroll-ELISA

Um die Nachweisgrenze konventioneller Verfahren zum Nachweis des PrPc mit den verwendeten Reagenzien zu Vergleichszwecken zu ermitteln wurde ein ELISA des Prion-Proteins PrPc durchgeführt.

Es wurden zunächst entsprechend Abschnitt 3.4. eine Verdünnungsreihe von PrPc in Proteinase K verdautem Rinderhirn-Homogenat vorbereitet. Im Anschluss wurden je 23,3 µl der PrPc-Lösungen in je 70 µl TBS-Puffer mit 2 ng/ml des biotinylierten Detektionsantikörpers aufgenommen und für eine Stunde bei 37°C in einem Eppendorf-Thermomixer bei 150 rpm inkubiert.

Zur Durchführung eines konventionellen Sandwich-Assays wurden Fänger-Antikörper beschichtete Mikrotiter-Module benötigt. Hierzu wurden Top-Yield Mikrotitermodule (Nunc) mit 30 µl je Kavität einer Lösung von 2,5 µg/ml des jeweiligen nicht-biotinylierten Antikörpers in RB-Puffer (50 mM Borsäure / NaOH, pH 9,5) für 12 Stunden bei 4°C inkubiert. Der Blockierungsschritt der Antikörperbeschichteten Module wurde entsprechend dem unter 3.1.1. geschilderten Blockierungsschritt für STV-beschichtete Module durchgeführt. Nach dem auf die Blockierung folgenden vierfachen Waschschritt mit TETBS wurden die Module für eine Stunde bei 37°C unter orbitalem Schütteln mit 30 µl / Kavität der Lösung von PrPc in Hirnhomogenat inkubiert.

Nach einem weiteren Standard-Waschschritt mit TETBS wurden die Module mit 30 µl / Kavität einer 1:5000 Verdünnung eines STV-Alkalische-Phosphatase-Konjugates (1000 U/ml vor Verdünnung, Roche) in TBS für eine Stunde bei Raumtemperatur unter orbitalem Schütteln inkubiert. Im Anschluss wurde ein weiterer Standard-TETBS-Waschschritt durchgeführt, bevor abschließend dreifach für je drei Minuten mit 220 µl / Kavität TBS gewaschen wurde. Nach Zugabe von 50 µl / Kavität des Fluoreszenz-generierenden Substrates AttoPhos™ (Roche) wurde die erhaltene Fluoreszenz mit einem VICTOR-Multilabelcounter (Wallac) gemessen.

3.7 Ergebnisse:

| **Zu quantifizierender Testansatz, enthaltend 10 pg PrPc in Hirn-Homogenat:** | |
|---|---|
| $O_T$: Signalintensität der Nachweis-Nucleinsäure im PCR-ELOSA (ohne Gegenwart von "KF-1" in der PCR) | 5750 a.u. |
| $N_T$:Signalintensität der Nachweis-Nucleinsäure im PCR-ELOSA: (2 fM "KF-1" in der PCR) | 3820 a.u. |
| $K_T$: Signalintensität der Kompetitor-Nucleinsäure im PCR-ELOSA: (2 fM"KF-1" in der PCR) | 2413 a.u. |
| **Negativkontrolle ohne PrPc** | |
| $O_N$: Signalintensität der Nachweis-Nucleinsäure im PCR-ELOSA (ohne Gegenwart von "KF-1" in der PCR) | 5112 a.u. |
| $N_N$:Signalintensität der Nachweis-Nucleinsäure im PCR-ELOSA: (2 fM "KF-1" in der PCR) | 2832 a.u. |
| $K_N$: Signalintensität der Kompetitor-Nucleinsäure im PCR-ELOSA; (2 fM "KF-1" in der PCR) | 3145 a.u. |
| Signal-zu-Hintergrundwert $S = O_T / O_N$ ohne "KF-1" | 1,1 |
| Signal-zu-Hintergrundwert $S = N_T / N_N$ in Gegenwart von "KF-1" | 1,3 |
| Vergleichsquotient $Q = (N_T / K_T) / (N_N / K_N)$: | 1,8 |

Der Vergleichsquotient Q, erhalten durch die Bildung der Teilquotienten aus den erhaltenen ELOSA-Werten für Nachweis- und Kompetitor-DNA, ergibt mit 1,8 einen deutlich höheren Wert für den 10 pg PrPc enthaltenden Testansatz als die normalerweise übliche Bestimmung des Signal-zu-Hintergrundwertes S.

Ohne Gegenwart der Kompetitor-Nucleinsäure wurde unter amsonsten identischen Bedingungen lediglich ein Signal-zu-Hintergrundwert von 1,1 für 10 pg PrPc in Himhomogenat erhalten. Mit einem konventionellen Kontroll-ELISA ist unter vergleichbaren Bedingungen kein PrPc unterhalb einer Menge von 100 pg PrPc in gespiktem Hirnhomogenat mehr nachweisbar.

**4. Ausführungsbeispiel: Nachweis von rekombinantem PrPc in Hirnhomogenat mittels eines Nachweis-Reagenzes unter Einsatz der Competitor-Nucleinsäure "KF-1"**

In diesem Ausführungsbeispiel wird ein aus der Literatur (C.M. Niemeyer et al., Nucleic Acids Res. 1999, 4553 bis 4561) bekanntes Verfahren zur Synthese eines in größeren Mengen herstell- und lagerbaren gereinigten Nachweis-Reagehs-Oligomers für PrPc eingesetzt. Bei der signalerzeugenden PCR-Amplifikation der in diesem Reagens erhaltenen Nachweis-Nucleinsäure "DNA-169" wurde die Kompetitor-Nucleinsäure "KF-1" zugesetzt.

4.1. Herstellung eines Nachweis-Reagens

Zur Herstellung eines gereinigten Nachweis-Reagens-Oligomers wurden 80 pmol des biotinylierten Antikörpers 6H4 wie in Niemeyer, Adler et al. (1999) beschrieben eingesetzt Nach Aufnahme von 1 µl des gereinigten Reagens-Oligomers in 3000 µl RDB wird die Lösung "DRO-2" erhalten.

4.2. Kupplung mit PrPc

Entsprechend Ausführungsbeispiel 3.4 wurde eine Verdünnungsreihe von PrPc in bovinem Hirngewebe-Homogwenat vorbereitet. Mikrotitermodule, die gemäß 3.6. mit Fänger-Antikörper beschichtet, geblockt und mit TETBS gewaschen waren, wurden im Anschluss mit 30 µl je Kavität der PrPc-haltigen Lösung für eine Stunde bei 37°C unter orbitalem Schütteln inkubiert. Nach der Durchführung eines Standard-Waschschrittes mit TETBS (siehe 3.1.2.) wurde mit 30 µl je Kavität der Lösung "DRO-2" (siehe 4.1.) für 30 Minunten unter orbitalem Schütteln bei 37°C inkubiert.

4.3. PCR

Die Durchführung der PCR unter Zugabe von 20 fM der Kompetitor-Nucleinsäure "KF-1" und des anschließenden ELOSAs erfolgte wie unter 3.5. beschrieben.

4.4. Ergebnis

| | |
|---|---|
| **Zu quantifizierender Testansatz, enthaltend 1 fg PrP in Hirn-Homogenat:** | |
| $N_T$:Signalintensität der Nachweis-Nucleinsäure im PCR-ELOSA: (20 fM "KF-1" in der PCR)<br>$K_T$: Signalintensität der Kompetitor-Nucleinsäure im PCR-ELOSA: (20 fM "KF-1" in der PCR) | 2476 a.u.<br>16586 a.u. |
| **Negativkontrolle ohne PrP:** | |
| $N_N$:Signalintensität der Nachweis-Nucleinsäure im PCR-ELOSA: (20 fM "KF-1" in der PCR)<br>$K_N$: Signalintensität der Kompetitor-Nucleinsäure im PCR-ELOSA: (20 fM "KF-1" in der PCR)<br>Signal-zu-Hintergrundwert $S = N_T / N_N$<br>Vergleichsquotient $Q = (N_T / K_T) / (N_N./ K_N)$: | 2013 a.u.<br>18409 a.u.<br>1,2<br>1,4 |

Der Vergleichsquotient Q, erhalten durch die Bildung der Teilquotienten aus den erhaltenen ELOSA-Werten für Nachweis- und Kompetitor-DNA, ergibt mit 1,4 einen deutlich höheren Wert für den 1 fg PrP enthaltenden Testansatz als die normalerweise übliche Bestimmung des Signal-zu-Hintergrundwertes S.

Ohne Gegenwart der Kompetitor-Nucleinsäure wurde unter amsonsten identischen Bedingungen ledich ein Signal-zu-Hintergrundwert von 1,1 für 1 fg PrP in Himhomogenat erhalten. Mit einem konventionellen Kontroll-ELISA ist unter vergleichbaren Bedingungen kein PrP unterhalb einer Menge von 100 pg PrP in gespikten Himhomogenat-Proben mehr nachweisbar.

**5. Ausführungsbeispiel: Nachweis von rekombinantem PrPc in Blutserum mittels eines Fänger- und eines Nachweis-Reagenzes unter Einsatz der Competitor-Nucleinsäure "KF-1"**

Der hochsensitive Nachweis von PrP in Blutproben ist zur in-vivo Diagnostik der TSE-Erkrankungen von besonderem Interesse. In diesem Ausführungsbeispiel wurde BISEKO, ein kommerziell erhältliches standardisiertes Humanserum (Biotest) als biologische Matrix eingesetzt. 1 ml des Serums wurde mit 10µl einer 10 mg/ml Proteinase K- Stammlösung (Roche) für 60 min bei 56°C inkubiert. Das Enzym wurde anschließend durch Zugabe von 100 µl einer 10 mg/ml Lösung von PMSF (Roche) in iso-Propanol inaktiviert. Diese BISEKO-Lösung wurde im Folgenden entsprechend dem Hirn-Homogenat des 3. Ausführungsbeispiels eingestzt. Der Nachweis von PrPc wurde entsprechend dem 3. Ausführungsbeispiel durchgeführt. Der PCR-Lösung wurden dabei 2fM der Kompetitor- Nucleinsäure "KF-1" zugesetzt.

Ergebnisse:

| **Zu quantifizierender Testansatz, enthaltend 10 pg PrPc in Serum:** | |
|---|---|
| $O_T$: Signalintensität der Nachweis-Nucleinsäure im PCR-ELOSA (ohne Gegenwart von "KF-1" in der PCR)<br>$N_T$:Signalintensität der Nachweis-Nucleinsäure im PCR-ELOSA: (2 fM "KF-1" in der PCR)<br>$K_T$: Signalintensität der Kompetitor-Nucleinsäure im PCR-ELOSA: (2 fM"KF-1" in der PCR) | 613 a.u.<br>619 a.u.<br>622 a.u. |
| **Negativkontrolle ohne PrPc** | |
| $O_N$: Signalintensität der Nachweis-Nucleinsäure im PCR-ELOSA (ohne Gegenwart von "KF-1" in der PCR)<br>$N_N$:Signalintensität der Nachweis-Nucleinsäure im PCR-ELOSA: (2 fM "KF-1" in der PCR)<br>$K_N$: Signalintensität der Kompetitor-Nucleinsäure im PCR-ELOSA: (2 fM "KF-1" in der PCR)<br>Signal-zu-Hintergrundwert S = $O_T$ / $O_N$ ohne "KF-1"<br>Signal-zu-Hintergrundwert S = $N_T$ / $N_N$ in Gegenwart von "KF-1"<br>Vergleichsquotient Q = ($N_T$ / $K_T$) / ($N_N$ / $K_N$): | 542 a.u.<br>526 a.u.<br>735 a.u.<br>1,1<br>1,2<br>1,4 |

Der Vergleichsquotient Q, erhalten durch die Bildung der Teilquotienten aus den erhaltenen ELOSA-Werten für Nachweis- und Kompetitor-DNA, ergibt mit 1,4 einen höheren Wert für den 10 pg PrPc enthaltenden Testansatz als die normalerweise übliche Bestimmung des Signal-zu-Hintergrundwertes S.

Ohne Gegenwart der Kompetitor-Nucleinsäure wurde unter amsonsten identischen Bedingungen lediglich ein Signal-zu-Hintergrundwert von 1,1 für 10 pg PrPc in Hirnhomogenat erhalten. Mit einem konventionellen Kontroll-ELISA ist unter vergleichbaren Bedingungen kein PrPc unterhalb einer Menge von 100 pg PrPc in gespiktem Serumproben mehr nachweisbar.

**6. Ausführungsbeispiel: : Nachweis von rekombinantem PrPc in Serum mittels eines Nachweis-Reagenzes unter Einsatz der Competitor-Nucleinsäure**

In diesem Ausführungsbeispiel wurde, entsprechend dem 5. Ausführungsbeispiel, Proteinase K behandeltes BISEKO als biologische Matrix eingesetzt. Diese BISEKO-Lösung wurde im Folgenden entsprechend dem Him-Homogenat des 4. Ausführungsbeispiels eingestzt. Der Nachweis von PrPc wurde entsprechend dem 4. Ausführungsbeispiel mit dem Reagenz "DRO-2" durchgeführt. Der PCR-Lösung wurden dabei 20fM der Kompetitor- Nucleinsäure "KF-1" zugesetzt.

Ergebnisse:

| **Zu quantifizierender Testansatz, enthaltend 1 fg PrP in Serum:** | |
|---|---|
| $N_T$:Signalintensität der Nachweis-Nucleinsäure im PCR-ELOSA: (20 fM "KF-1" in der PCR)<br>$K_T$: Signalintensität der Kompetitor-Nucleinsäure im PCR-ELOSA: (20 fM "KF-1" in der PCR) | 130 a.u.<br>1041 a.u. |
| **Negativkontrolle ohne PrP:** | |
| $N_N$:Signalintensität der Nachweis-Nudeinsäure im PCR-ELOSA: (20 fM "KF-1" in der PCR)<br>$K_N$: Signalintensität der Kompetitor-Nucleinsäure im PCR-ELOSA: (20 fM "KF-1" in der PCR)<br>Signal-zu-Hintergrundwert S = $N_T$ / $N_N$ | 110 a.u.<br>1130 a.u.<br>1,2 |

(fortgesetzt)

| **Negativkontrolle ohne PrP:** | |
|---|---|
| Vergleichsquotient Q = $(N_T / K_T) / (N_N / K_N)$: | 1,3 |

Der Vergleichsquotient Q, erhalten durch die Bildung der Teilquotienten aus den erhaltenen ELOSA-Werten für Nachweis- und Kompetitor-DNA, ergibt mit 1,3 einen höheren Wert für den 1 fg PrP enthaltenden Testansatz als die normalerweise übliche Bestimmung des Signal-zu-Hintergrundwertes S.

Ohne Gegenwart der Kompetitor-Nucleinsäure wurde unter amsonsten identischen Bedingungen ledich ein Signal-zu-Hintergrundwert von 1 für 1 fg PrP erhalten. Mit einem konventionellen Kontroll-ELISA ist unter vergleichbaren Bedingungen kein PrP unterhalb einer Menge von 100 pg PrP in gespikten Serum-Proben mehr nachweisbar.

**7. Ausführungsbeispiel: Nachweis von rViscumin (rekombinantem Mistel-Lektin) unter Einsatz einer Kompetitor-Nucleinsäure**

rViscumin, ein rekombinant aus den genetischen Informationen des Mistel-Lektins abgeleiteter neuer pharmakologischer Wirkstoff, hat sich im Tierversuch auch in sehr geringen Dosierungen als hochwirksames Anti-Tumormittel erwiesen (siehe dazu auch Eck, J., M. Langer, B. Mockel, A. Baur, M. Rothe, H. Zinke und H. Lentzen (1999), "Cloning of the mistletoe lectin gene and characterization of the recombinant A-chain." *Eur J Biochem* **264**(3); 775-84 sowie Eck, J., M. Langer, B. Mockel, K. Witthohn, H. Zinke und H. Lentzen (1999), "Characterization of recombinant and plant-derived mistletoe lectin and their B-chains." *Eur J Biochem* **265**(2); 788-97). Zur weitergehenden Untersuchung insbesondere der pharmakokinetischen und toxikokinetischen Eigenschaften des rViscumins ist ein extrem sensitives Nachweisverfahren von großem Interesse.

7.1. Herstellung eines Nachweis-Reagenz-Oligomer

Für den Nachweis des rViscumins (Viscum AG), wurden entsprechend des bereits im 4. Ausführungsbeispiel zum Nachweis des PrPc verwendeten, aus der Literatur bekannten Verfahrens (C.M. Niemeyer et al., Nucleic Acids Res. 1999, 4553 bis 4561) Nachweis-Reagens-Oligomere mit dem biotinylierten polyclonalen Kaninchen-anti-rViscum Antikörper T2b ("Tier 2", #2758g, 1,2 mg/ml, ca. 8 pmol/µl, 2,7 mol Biotin / 1 mol Antikörper, BRAIN AG) hergestellt. Dabei wurden 75 pmol T2b anstelle des Anti-PrPc-Antikörpers eingesetzt.

7.2. Herstellung Fänger-Antikörper beschichteter Mikrotitermodule

Die Herstellung Fänger-Antikörper beschichteter TopYield-Module (Nunc) zur Durchführung eines Sandwich-Assays erfolgte unter Verwendung des nicht-biotinylierten Antikörpers "T2" ("Tier 2", #2578g, BRAIN AG) entsprechend Ausführungsbeispiel 3.6. Die Module wurden mit 30 µl je Kavität einer 5 µg/ml Lösung des Antikörpers "T2" beschichtet.

7.3. Kupplung mit rViscumin und anschließender Nachweis

Das Antigen rViscumin (Viscum AG) wurde mit einer Konzentration von 2 ng/µl in Aliquoten zu je 10 µl bei -80°C gelagert und erst unmittelbar vor Gebrauch aufgetaut. Die Verdünnung des rViscumins auf Konzentrationen zwischen 2 ng/ml und 200 fg/ml erfolgte in BISEKO, einem virusinaktivierten standardisierten Humanserum der Firma Biotest, um die Assaybedingungen eines rViscumin-Nachweises aus Patientenserum zu simulieren. Als Negativkontrolle wurde reines BISEKO verwendet. 30 µl der rViscumin-haltigen Lösungen wurden für 30 min bei Raumtemperatur in Fänger-Antikörper beschichteten Modulen (siehe 7.2.) inkubiert. Die Module wurden einem Standard-Waschschritt mit TETBS unterzogen (siehe 3.1.2.) und im Anschluß gemäß dem aus der Literatur bekannten Protokoll (C.M. Niemeyer et al., Nucleic Acids Res. 1999, 4553 bis 4561) mit dem antirViscumin-Nachweis-Reagens-Oligomer gekuppelt. Die Durchführung des anschließenden PCR-Schrittes erfolgte wie unter 3.5. beschrieben. Dabei wurde in zwei getrennten Experimenten sowohl die Kompetitor-Nucleinsäure "KF-1" als auch die Kompetitor-Nucleinsäure "KF-2" in einer Menge von $1x10^{-20}$ mol "KF-1" beziehungsweise $1x10^{-20}$ mol "KF-2" pro PCR-Ansatz zugegeben. Zur Amplifikation des PCR-Ansatzes mit "KF-1" wurden die Primer "N-1" und "N-2" eingesetzt, bei Verwendung von "KF-2" wurden zusätzlich die Primer "H-1" und "H-2" verwendet (Sequenzen siehe Anhang). Der ELOSA-Nachweis der Amplifikate erfolgte wie unter 1.4. beschrieben, als Fänger-Oligonucleotid für "KF-2" wurde die 5'biotinylierte Sequenz "H-1". als Fänger-Oligonucleotid, für "KP-1" sowie die Nachweis-Nucleinsäure "DNA- 169" wurde "cK-Es" respektive "cN-Es" eingesetzt.

7.4. Kontrollexperimente

Es wurde zusätzlich ein Assay ohne Zugabe der Kompetitor-Nucleinsäure sowie entsprechend Ausführungsbeispiel 3.6. ein Kontroll-ELISA unter Verwendung des biotinylierten Antikörpers "T2b" (siehe 7.1) und einem Streptavidin-alkalische-Phosphatase-Konjugat durchgeführt.

**7.5. Ergebnisse**

*a.) Verwendung der Kompetitor-Nucleinsäure "KF-2":*

| **Zu quantifizierender Testansatz, enthaltend 1 attomol (ca. 2 pg/ml) rViscumin:** | |
|---|---|
| $O_T$: Signalintensität der Nachweis-Nucleinsäure im PCR-ELOSA (ohne Gegenwart von "KF-1" oder "KF-2" in der PCR) | 42460 a.u. |
| $N_T$:Signalintensität der Nachweis-Nucleinsäure im PCR-ELOSA: (1x$10^{-20}$ mol "KF-2" in der PCR) | 24426 a.u. |
| $K_T$: Signalintensität der Kompetitor-Nucleinsäure im PCR-ELOSA: (1x$10^{-20}$ mol "KF-2" in der PCR) | 6381 a.u. |
| **Negativkontrolle ohne rViscumin:** | |
| $O_N$: Signalintensät der Nachweis-Nucleinsäure im PCR-ELOSA (ohne Gegenwart von "KF-1" oder "KF-2" in der PCR) | 33224 a.u. |
| NN:Signalintensität der Nachweis-Nucleinsäure im PCR-ELOSA: (1x$10^{-20}$ mol "KF-2" in der PCR) | 18253 a.u. |
| $K_N$: Signalintensität der Kompetitor-Nucleinsäure im PCR-ELOSA: (1x$10^{-20}$ mol "KF-2" in der PCR) | 8264 a.u. |
| Signal-zu-Hintergrundwert S = $O_T/O_N$ ohne "KF-2" oder "KF-1" | 1,3 |
| Signal-zu-Hintergrundwert S = $N_T$ / $N_N$ in Gegenwart von "KF-2" | 1,3 |
| Vergleichsquotient Q = ($N_T$ / $K_T$) / ($N_N$ / $K_N$): | 1,7 |

Der Vergleichsquotient Q, erhalten durch die Bildung der Teilquotienten aus den erhaltenen ELOSA-Werten für Nachweis- und Kompetitor-DNA, ergibt mit 1,7 einen deutlich höheren Wert für den 1 attomol rViscumin enthaltenden Testansatz als die normalerweise übliche Bestimmung des Signal-zu-Hintergrundwertes S. Ohne Gegenwart der Kompetitor-Nucleinsäure wurde unter identischen Bedingungen lediglich ein Signal-zu-Hintergrundwert von 1,3 für 1 attomol rViscumin erhalten. Mit einem konventionellen ELISA ist unter vergleichbaren Bedingungen kein rViscumin unterhalb einer Menge von 10 attomol in 30 µl BISEKO (ca. 20 pg/ml) mehr nachweisbar.

*b.) Verwendung der Kompetitor-Nucleinsäure "KF-1"*

| **Zu quantifizierender Testansatz, enthaltend 0,1 attomol rViscumin in Serum:** | |
|---|---|
| $O_T$: Signalintensität der Nachweis-Nucleinsäure im PCR-ELOSA (ohne Gegenwart von "KF-1" oder "KF-2" in der PCR) | 35286 a.u. |
| $N_T$:Signalintensität der Nachweis-Nucleinsäure im PCR-ELOSA: (1x$10^{-20}$ mol "KF-1" in der PCR) | 16304 a.u. |
| $K_T$: Signalintensität der Kompetitor-Nucleinsäure im PCR-ELOSA: (1x$10^{-20}$ mol "KF-1" in der PCR) | 2987 a.u. |
| **Negativkontrolle ohne rViscumin:** | |
| $O_N$: Signalintensität der Nachweis-Nucleinsäure im PCR-ELOSA (ohne Gegenwart von "KF-1" oder "KF-2" in der PCR) | 33224 a.u. |
| $N_N$:Signalintensität der Nachweis-Nucleinsäure im PCR-ELOSA: (1x$10^{-20}$ mol "KF-1" in der PCR) | 11961 a.u. |
| $K_N$: Signalintensität der Kompetitor-Nucleinsäure im PCR-ELOSA: (1x$10^{-20}$ mol "KF-1" in der PCR) | 4043 a.u. |
| Signal-zu-Hintergrundwert S = $O_T$ / $O_N$ ohne "KF-2" oder "KF-1" | 1,1 |

(fortgesetzt)

| **Negativkontrolle ohne rViscumin:** | |
|---|---|
| Signal-zu-Hintergrundwert $S = N_T / N_N$ in Gegenwart von "KF-1"<br><br>Vergleichsquotient $Q = (N_T / K_T) / (N_N / K_N)$: | <u>1,4</u><br><br><u>1,8</u> |

Der Vergleichsquotient Q, erhalten durch die Bildung der Teilquotienten aus den erhaltenen ELOSA-Werten für Nachweis- und Kompetitor-DNA, ergibt mit 1,8 einen deutlich höheren Wert für den 0,1 attomol rViscumin enthaltenden Testansatz als die normalerweise übliche Bestimmung des Signal-zu-Hintergrundwertes S. Ohne Gegenwart der Kompetitor-Nucleinsäure wurde unter identischen Bedingungen lediglich ein Signal-zu-Hintergrundwert von 1,1 für 0,1 attomol rViscumin erhalten. Mit einem ELISA ist unter vergleichbaren Bedingungen kein rViscumin unterhalb einer Menge von 10 attomol in 30 µl BISEKO (ca. 20 pg/ml) mehr nachweisbar.

c.) Unter Verwendung der sensitiveren Kombination aus der Nachweis-Nucleinsäure "DNA-169" und der Kompetitor-Nucleinsäure "KF-1" konnten 0,1 pg/ml rViscumin nachgewiesen werden (siehe Fig. 4). Dabei wurde in einem Bereich von 4 Dekaden eine lineare Abhängigkeit zwischen Q und den eingesetzten Konzentrationen c beobachtet.

<u>**Anhang: Nucleinsäuresequenzen**</u>

**1. Primer N-1**

5'-AGC GGA TAA CAA TTT CAC ACA GGA-3'

**2. Primer N-2**

5'-AAG GCG ATT AAG TTG GAA AAC-3'

**3. Primer K-1**

5'- AGC GGA TAA CAA TTT CAC ACA GGA CGC TCA TGA GAC ATA ACC CTG-3'

**4. Primer K-2**

ATG CCG CAA AAA AGG G-3'

**5. Primer H-1**

5'- TGC AGC CTC CAG GAC C -3'

**6. Primer H-2**

5´-GCA CTC GCA AGC ACC CTA -3´

**7. Fänger-Oligonucleotid cN-Es**

Biotin-5´-GTA ATC ATG GTC ATA GCT GTT -3´

**8. Fänger-Oligonucleotld cK-Es**

Biotin-5´-ACT ATC CCA CGA ACG CTC A -3´

**9. Kupplungs-Sequenz bcA**

Biotin-5´-ACT ATC CCA CGA ACG CTC A -3´

**10. Kupplunge-Sequenz A**

5'-TCC TGT GTG AAA TTG TTA TCC GCT-3'

**11 Erkennungssequenz N-Es**

5´-AAC AGC TAT GAC CAT GAT TAC -3´

**12 Erkennungssequenz K-Es**

5´-TGA GCG TTC GTG GGA TAG T -3´

**13. Nachweis-Nucleinsäure ("DNA-169" - Position 6252-6354 auf M13mp18):**

```
                  10         20         30         40         50
Biotin-5'- AAGGCGATTA AGTTGGGTAA CGCCAGGGTT TTCCCAGTCA CGACGTTGTA
                  60         70         80         90        100
           AAACGACGGC CAGTGCCAAG CTTGCATGCC TGCAGGTCGA CTCTAGAGGA
                 110        120        130        140        150
           TCCCCGGGTA CCGAGCTCGA ATTCGTAATC ATGGTCATAG CTGTTTCCTG
                 160        169
           TGTGAAATTG TTATCCGCT -3'-Biotin
```

Die Lage der Bindungssequenz für den PCR-Primer "N-2" ist einfach unterstrichen, die Lage der Bindungssequenz für den Primer "N-1" ist doppelt unterstrichen, die Lage der Erkennungssequenz "N-Es" ist **fett und unterstrichen** hervorgehoben.

**14. Kompetitor-Nucleinsäure ("KF1" - artifiziell) mit den Primerbindungsstellen "N-2" und "N-1" der Nachweis-Nucleinsäure:**

```
           10         20         30         40         50
5'-AAGGCGATTA AGTTGGGTAA CGCCATGAGC GTTCGTGGGA TAGTCAAAAT
           60         70         80         90        100
   GCCGCAAAAA AGGGAATAAG GGCGACACGG AAATGTTGAA TACTCATACT
          110        120        130        140        150
   CTTCCTTTTT CAATATTATT GAAGCATTTA TCAGGGTTAT TGTCTCATGA
          160        170    177
   GCGTCCTGTG TGAAATTGTT ATCCGCT-3'
```

Die Lage der Bindungssequenz für den PCR-Primer "N-2" ist einfach unterstrichen, die Lage der Bindungssequenz für den Primer "N-1" ist doppelt unterstrichen, die Lage der Erkennungssequenz "K-Es" ist **fett und unterstrichen** hervorgehoben.

**15. Kompetitor-Nucleinsäure ("KF-2",)**

```
           10         20         30         40         50
5'-TGCAGCCTCC AGGACCCCCC CTCCCGGGAG AGCCATAGTG GTCTGCGGAA
           60         70         80         90        100
   CCGGTGAGTA CACCGGAATT GCCAGGACGA CCGGGTCCTT TCTTGGATTA
          110        120        130        140        150
   ACCCGCTCAA TGCCTGGAGA TTTGGGCGTG CCCCCGCGAG ACTGCTAGCC
          160        170        180        190        200
   GAGTAGTGTT GGGTCGCGAA AGGCCTTGTG GTACTGCCTG ATAGGGTGCT
          209
   TGCGAGTGC-3'
```

Die Lage der Bindungssequenz für den PCR-Primer "H-1" ist einfach unterstrichen, die Lage der Bindungssequenz für den Primer "H-2" ist doppelt unterstrichen, als Erkennungssequenz wird ebenfalls der Sequenzabschnitt "H-1" verwendet.

## Patentansprüche

1. Verfahren zum Nachweisen einer Substanz in einem Testansatz, umfassend die Schritte:

   a) Bilden eines Nachweiskomplexes im Testansatz, wobei im Nachweiskomplex

   - die nachzuweisende Substanz an ein Nachweis-Bindungsreagens gebunden ist und
   - das Nachweis-Bindungsreagens mit einer Nachweis-Nucleinsäure, die mit einem Replikations-Reagens replizierbar ist, zu einem Nachweiskonjugat verbunden ist,

   b) Zugeben einer von der Nachweis-Nucleinsäure unterscheidbaren Kompetitor-Nucleinsäure zum Testansatz, wobei die Kompetitor-Nucleinsäure ebenfalls durch das Replikations-Reagens replizierbar ist und mit der Nachweis-Nucleinsäure um einen oder mehrere Bestandteile des Replikations-Reagens konkurriert,
   c) Kontaktieren der Nachweis- und Kompetitor-Nucleinsäuren mit dem Replikations-Reagens zum Replizieren der Nachweis- und Kompetitor-Nucleinsäuren,
   d) Nachweisen der replizierten Nachweis-Nucleinsäure und der replizierten Kompetitor-Nucleinsäure.

2. Verfahren nach Anspruch 1,
   **dadurch gekennzeichnet, dass** der in Schritt a) gebildete Nachweis-Komplex:.

   - zwei oder mehr miteinander verbundene Nachweis-Bindungsreagenzien um-fasst und/oder
   - zwei oder mehr Nachweis-Nucleinsäuren umfasst.

3. Verfahren nach einem der vorherigen Ansprüche,
   **dadurch gekennzeichnet, dass** in einem weiteren Schritt e) die nachzuweisende Substanz an einer Oberfläche immobilisiert wird.

4. Verfahren nach Anspruch 3,
   **dadurch gekennzeichnet, dass** zur Durchführung von Schritt e)

   - die nachzuweisende Substanz an ein Immobilisierungs-Bindungsreagens gebunden ist, wobei das Immobilisierungs-Bindungsreagens mit einer ersten Immobilisierungs-Nucleinsäure verbunden ist (Fängerkonjugat), und
   - die erste Immobilisierungs-Nucleinsäure mit einer an einer Oberfläche immobilisierten zweiten Immobilisierungs-Nucleinsäure hybridisiert wird, um das Immobilisierungs-Bindungsreagens an der Oberfläche zu immobilisieren.

5. Verfahren nach einem der vorherigen Ansprüche,
   **dadurch gekennzeichnet, dass** zusätzlich zu einem Testansatz in einem weiteren Ansatz (Negativkontrolle)

   f) das Replikations-Reagens ohne die nachzuweisende Substanz unter den Bedingungen behandelt wird, wie sie für eine Nucleinsäure-Replikation der Nachweis- und der Kompetitor-Nucleinsäuren benötigt werden,
   g) die Menge der in der Negativkontrolle gegebenenfalls replizierten Nucleinsäuren bestimmt wird und
   h) die Mengen replizierter Nachweis- und Kompetitor-Nudeinsäuren mit der Menge der in der Negativkontrolle gebildeten Nucleinsäuren verglichen werden.

6. Verfahren nach einem der vorherigen Ansprüche,
   **dadurch gekennzeichnet, dass** die Nachweis- und Kompetitor-Nucleinsäuren eine Länge von 50 bis 700 Nucleobasen besitzen.

7. Verfahren nach einem der vorherigen Ansprüche,
   **dadurch gekennzeichnet, dass** die nachzuweisende Substanz bei Durchführung des erfindungsgemäßen Nachweisverfahrens in einer biologischen Matrix vorliegt.

8. Verfahren nach einem der vorherigen Ansprüche,
   **dadurch gekennzeichnet, dass** die nachzuweisende Substanz ein Prion-Protein oder ein Lektin, vorzugsweise ein Mistel-Lektin, ist.

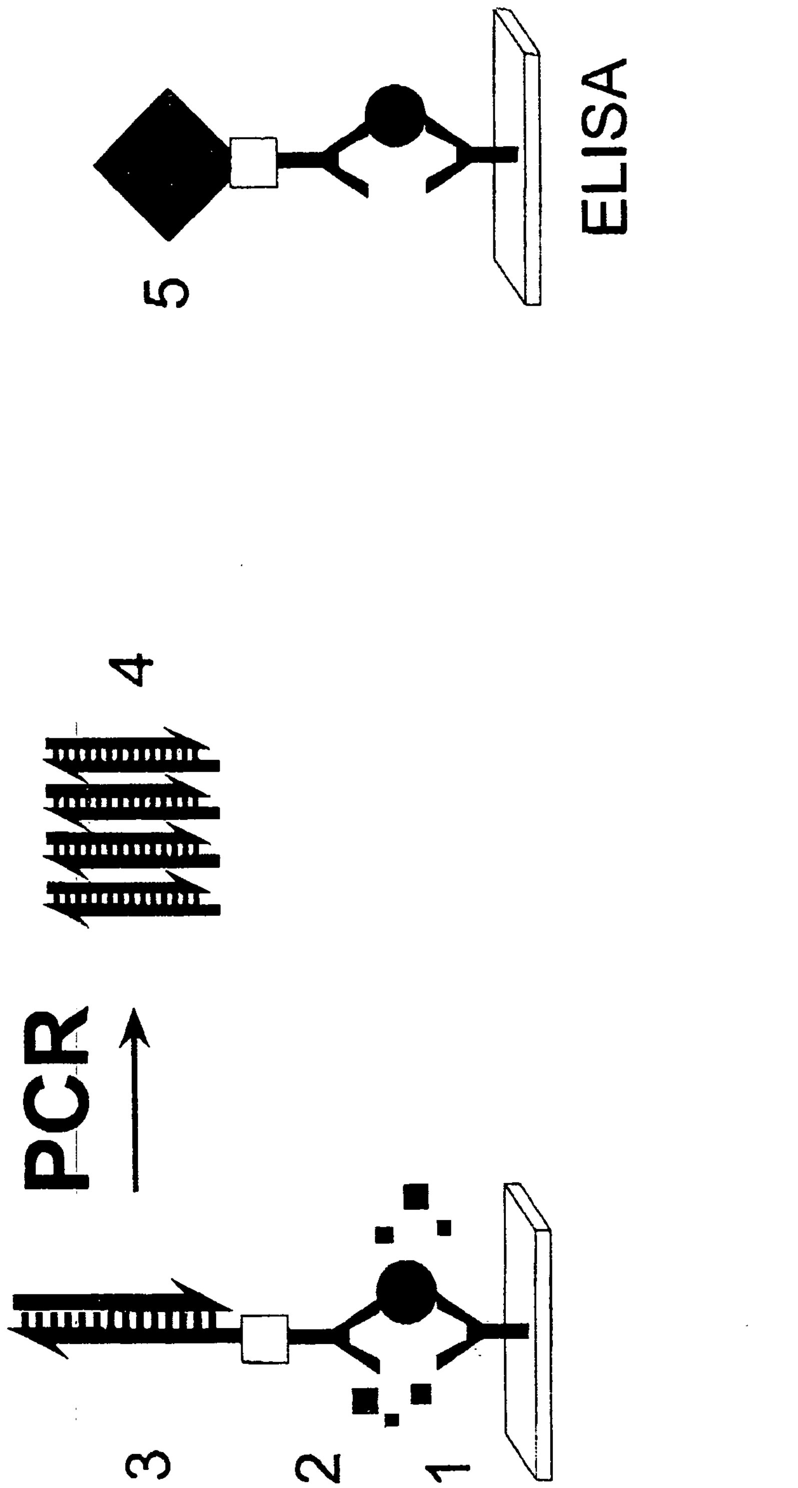


Fig. 1

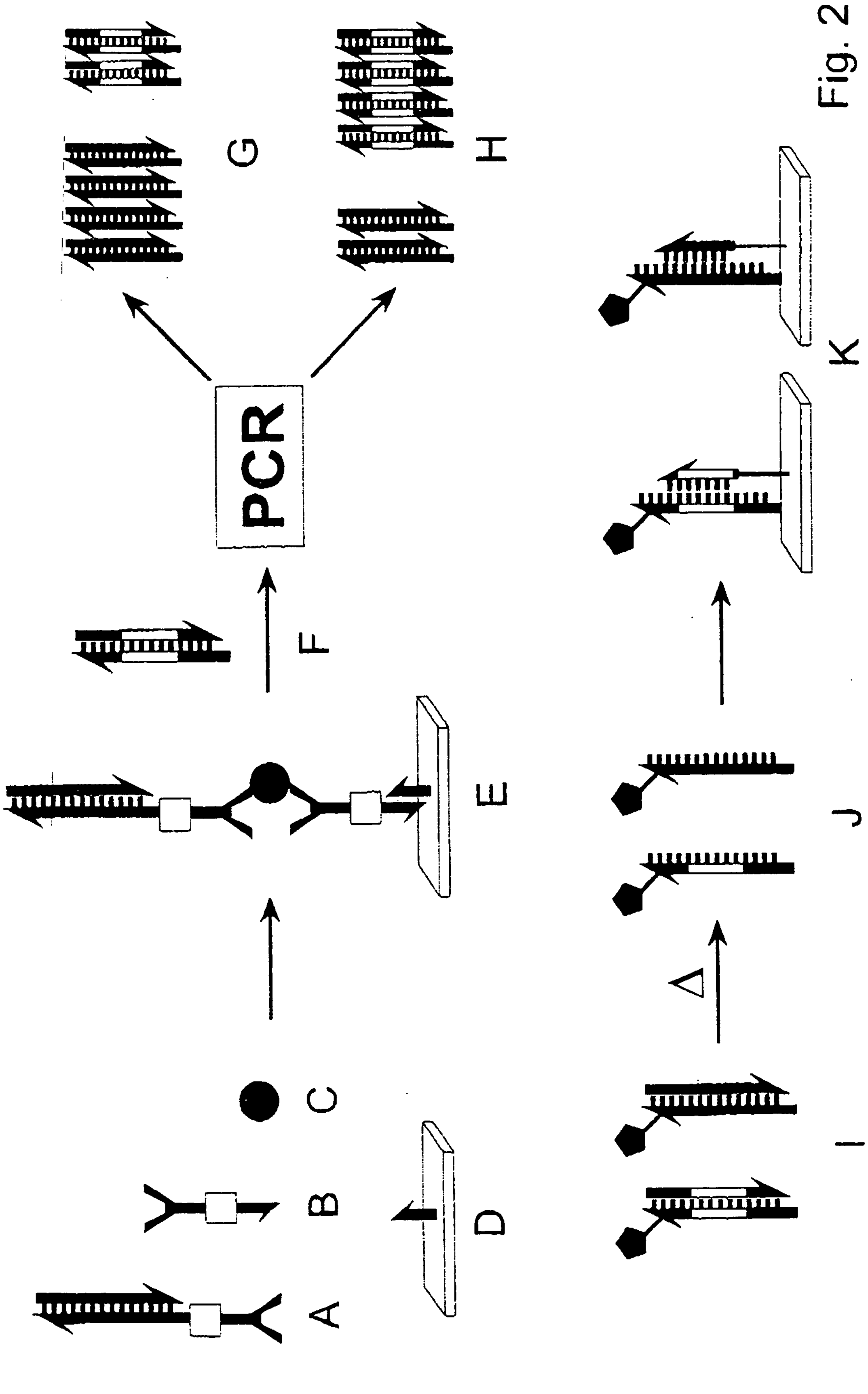


Fig. 2

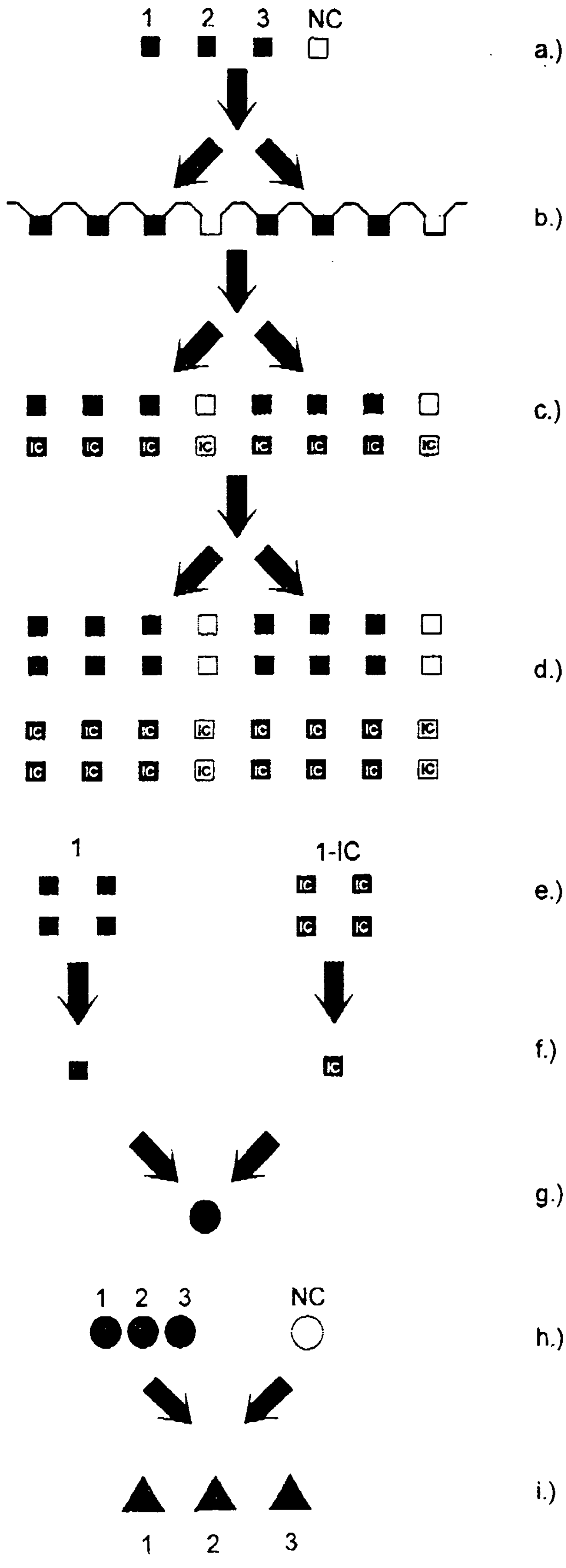


Fig. 3

A:

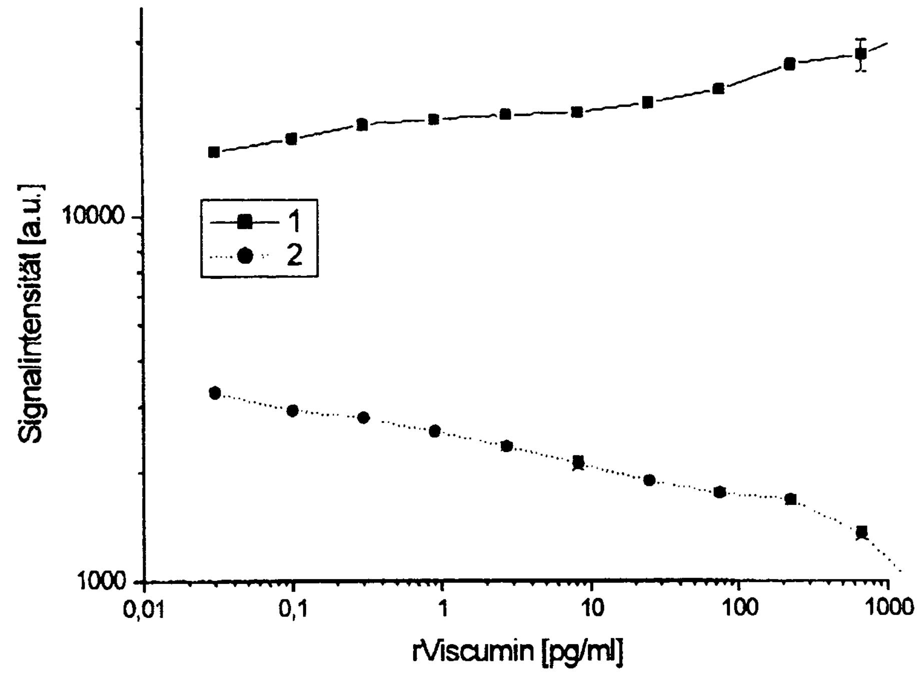
Signalintensität [a.u.]
10000
1000
0,01
0,1
1
10
100
1000
rViscumin [pg/ml]
1
2

B:

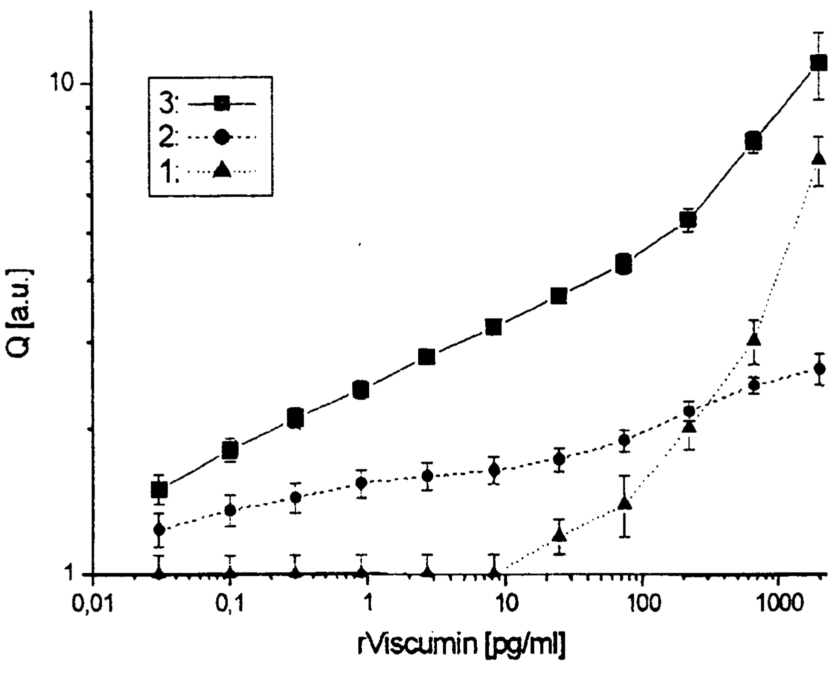
Q [a.u.]
10
1
0,01
0,1
1
10
100
1000
rViscumin [pg/ml]
3:
2:
1:

Fig. 4

Europäisches Patentamt

# EUROPÄISCHER RECHERCHENBERICHT

Nummer der Anmeldung
EP 01 11 4562

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| X<br><br><br>Y | WO 99 63109 A (BAXTER AG ;TURECEK PETER (AT); SCHWARZ HANS PETER (AT); SIEKMANN J) 9. Dezember 1999 (1999-12-09)<br>* das ganze Dokument *<br>--- | 1,3,5-8<br><br><br>2,4 | C12Q1/68<br>G01N33/53 |
| X | WO 98 04745 A (SINAI SCHOOL MEDICINE) 5. Februar 1998 (1998-02-05)<br>* das ganze Dokument *<br>--- | 1,3,5-7 | |
| Y | DE 199 41 756 A (NIEMEYER CHRISTOF) 8. März 2001 (2001-03-08)<br>* das ganze Dokument *<br>--- | 1-8 | |
| D,Y | NIEMEYER CHRISTOF M ET AL: "Self-assembly of DNA-streptavidin nanostructures and their use as reagents in immuno-PCR" NUCLEIC ACIDS RESEARCH, OXFORD UNIVERSITY PRESS, SURREY, GB, Bd. 27, Nr. 23, 1. Dezember 1999 (1999-12-01), Seiten 4553-4561, XP002176567 ISSN: 0305-1048<br>* das ganze Dokument *<br>--- | 1-8 | RECHERCHIERTE SACHGEBIETE (Int.Cl.7)<br>C12Q |
| D,Y | NIEMEYER CHRISTOF M ET AL: "Nanostructured DNA-protein aggregates consisting of covalent oligonucleotide-streptavidin conjugates." BIOCONJUGATE CHEMISTRY, Bd. 12, Nr. 3, Mai 2001 (2001-05), Seiten 364-371, XP002187380 ISSN: 1043-1802<br>* das ganze Dokument *<br>---<br>-/-- | 1-8 | |

Der vorliegende Recherchenbericht wurde für alle Patentansprüche erstellt

| Recherchenort | Abschlußdatum der Recherche | Prüfer |
|---|---|---|
| DEN HAAG | 15. Januar 2002 | Hagenmaier, S |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

Europäisches Patentamt

# EUROPÄISCHER RECHERCHENBERICHT

Nummer der Anmeldung

EP 01 11 4562

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| Y | NIEMEYER CHRISTOF M ET AL: "Fluorometric polymerase chain reaction (PCR) enzyme-linked immunosorbent assay for quantification of immuno-PCR products in microplates" ANALYTICAL BIOCHEMISTRY, ACADEMIC PRESS, SAN DIEGO, CA, US, Bd. 246, Nr. 1, 1997, Seiten 140-145, XP002151884 ISSN: 0003-2697 * das ganze Dokument * --- | 1-8 | |
| Y | HENDRICKSON EDWIN R ET AL: "High sensitivity multianalyte immunoassay using covalent DNA-labeled antibodies and polymerase chain reaction" NUCLEIC ACIDS RESEARCH, OXFORD UNIVERSITY PRESS, SURREY, GB, Bd. 23, Nr. 3, 1995, Seiten 522-529, XP002151883 ISSN: 0305-1048 * das ganze Dokument * --- | 1-8 | RECHERCHIERTE SACHGEBIETE (Int.Cl.7) |
| Y | CASE M C ET AL: "Enhanced ultrasensitive detection of structurally diverse antigens using a single immuno-PCR assay protocol" JOURNAL OF IMMUNOLOGICAL METHODS, ELSEVIER SCIENCE PUBLISHERS B.V.,AMSTERDAM, NL, Bd. 223, Nr. 1, 1. Februar 1999 (1999-02-01), Seiten 93-106, XP004155757 ISSN: 0022-1759 * das ganze Dokument * --- -/-- | 1-8 | |

Der vorliegende Recherchenbericht wurde für alle Patentansprüche erstellt

| Recherchenort | Abschlußdatum der Recherche | Prüfer |
|---|---|---|
| DEN HAAG | 15. Januar 2002 | Hagenmaier, S |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

Europäisches Patentamt

# EUROPÄISCHER RECHERCHENBERICHT

Nummer der Anmeldung
EP 01 11 4562

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| D,Y | NIEMEYER CHRISTOF M ET AL: "DNA-directed immobilization: Efficient, reversible, and site-selective surface binding of proteins by means of covalent DNA-streptavidin conjugates" ANALYTICAL BIOCHEMISTRY, ACADEMIC PRESS, SAN DIEGO, CA, US, Bd. 268, Nr. 1, 1. März 1999 (1999-03-01), Seiten 54-63, XP002176566 ISSN: 0003-2697 * das ganze Dokument * --- | 4 | |
| Y | NIEMEYER CHRISTOF M ET AL: "Functionalization of covalent DNA-streptavidin conjugates by means of biotinylated modulator components." BIOCONJUGATE CHEMISTRY, Bd. 10, Nr. 5, 1999, Seiten 708-719, XP002187381 ISSN: 1043-1802 * das ganze Dokument * --- | 4 | RECHERCHIERTE SACHGEBIETE (Int.Cl.7) |
| Y | NIEMEYER C M ET AL: "HYBRIDIZATION CHARACTERISTICS OF BIOMOLECULAR ADAPTORS, COVALENT DNA-STREPTAVIDIN CONJUGATES" BIOCONJUGATE CHEMISTRY, AMERICAN CHEMICAL SOCIETY, WASHINGTON, US, Bd. 9, Nr. 2, 1. März 1998 (1998-03-01), Seiten 168-175, XP000750892 ISSN: 1043-1802 * das ganze Dokument * --- | 4 | |
| Y | REISCHL U ET AL: "QUANTITATIVE PCR A SURVEY OF THE PRESENT TECHNOLOGY" MOLECULAR BIOTECHNOLOGY, TOTOWA, NJ, US, Bd. 3, 1995, Seiten 55-71, XP000600241 ISSN: 1073-6085 * das ganze Dokument * ----- | 1-8 | |

Der vorliegende Recherchenbericht wurde für alle Patentansprüche erstellt

| Recherchenort | Abschlußdatum der Recherche | Prüfer |
|---|---|---|
| DEN HAAG | 15. Januar 2002 | Hagenmaier, S |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

**ANHANG ZUM EUROPÄISCHEN RECHERCHENBERICHT ÜBER DIE EUROPÄISCHE PATENTANMELDUNG NR.** EP 01 11 4562

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

15-01-2002

| Im Recherchenbericht angeführtes Patentdokument | Datum der Veröffentlichung | Mitglied(er) der Patentfamilie | Datum der Veröffentlichung |
|---|---|---|---|
| WO 9963109 A | 09-12-1999 | AT 407160 B | 25-01-2001 |
| | | AT 96398 A | 15-05-2000 |
| | | WO 9963109 A1 | 09-12-1999 |
| | | AU 4022699 A | 20-12-1999 |
| | | EP 1082464 A1 | 14-03-2001 |
| WO 9804745 A | 05-02-1998 | US 5876924 A | 02-03-1999 |
| | | EP 1007728 A1 | 14-06-2000 |
| | | JP 2001521373 T | 06-11-2001 |
| | | WO 9804745 A1 | 05-02-1998 |
| DE 19941756 A | 08-03-2001 | DE 19941756 A1 | 08-03-2001 |

EPO FORM P0461

Für nähere Einzelheiten zu diesem Anhang : siehe Amtsblatt des Europäischen Patentamts, Nr.12/82